# EUROPÄISCHE PATENTSCHRIFT

(11) **EP 3 258 909 B1**
(45) Veröffentlichungstag und Bekanntmachung des Hinweises auf die Patenterteilung: **25.09.2019**
(21) Anmeldenummer: 16704562.4
(22) Anmeldetag: 09.02.2016
(51) Int. Cl.: A61K 8/37, A61K 9/127, A61K 8/00, C07C 69/675, C11D 3/16, C11D 17/00

(54) **2,3,4,5-TETRAHYDROXYHEXANDISÄUREESTER AUS DILACTONEN DER 2,3,4,5-TETRAHYDROXYHEXANDISÄURE**
2,3,4,5-TETRAHYDROXY ADIPIC ACID ESTERS FROM TETRAHYDROXY ADIPIC ACID DILACTONES
ESTER D'ACIDE 2,3,4,5-TRIHYDROXYHEXANEDIOÏQUE PROVENANT DE DILACTONE D'ACIDE TRIHYDROXYHEXANEDIOÏQUE

(30) Priorität: 19.02.2015 EP 15155806
(43) Veröffentlichungstag der Anmeldung: 27.12.2017
(73) Patentinhaber: BASF SE, 67056 Ludwigshafen (DE)
(72) Erfinder: OHLMANN, Dominik, 68163 Mannheim (DE); DIERKER, Markus, 40593 Düsseldorf (DE); BUSCH, Stefan, 40597 Düsseldorf (DE); PIETSCH, Oliver, 45481 Mülheim (DE)
(74) Vertreter: BASF IP Association
(86) Internationale Anmeldenummer: PCT/EP2016/052678
(87) Internationale Veröffentlichungsnummer: WO 2016/131672

(56) Entgegenhaltungen:
- EP-A1- 0 526 301
- CARPENTER CHRISSIE A ET AL: "Modifications in the nitric acid oxidation ofd-mannose: X-ray crystal structure ofN,N'-dimethyld-mannara", CARBOHYDRATE RESEARCH, PERGAMON, GB, Bd. 376, 16. Mai 2013 (2013-05-16), Seiten 29-36, XP028571342, ISSN: 0008-6215, DOI: 10.1016/J.CARRES.2013.05.004 in der Anmeldung erwähnt

## Beschreibung

### HINTERGRUND DER ERFINDUNG

Die vorliegende Erfindung betrifft ein Verfahren zur Herstellung von 2,3,4,5-Tetrahydroxyhexandisäureestern durch Veresterung der entsprechenden Dilactone der 2,3,4,5-Tetrahydroxyhexandisäure mit Alkoholen, die nach diesem Verfahren erhältlichen neuen 2,3,4,5-Tetrahydroxyhexandisäureester sowie deren Verwendung als grenzflächenaktive Verbindung.

### STAND DER TECHNIK

Grenzflächenaktive Verbindungen (englisch surfactant = surface active agent) haben in der Technik äußerst breite Verwendung gefunden. Je nach ihrer chemischen Zusammensetzung und Anwendung werden grenzflächenaktive Substanzen als Tenside (Detergenzien, Seife), Emulgatoren oder Netzmittel bezeichnet, in der Medizin (Physiologie) auch als Surfactant. Bezüglich ihrer primären Wirkung werden grenzflächenaktive Verbindungen speziell im Bereich Personal care und Home care auch in Tenside, Emulgatoren und Schaumbildner unterschieden.

Unter Tensiden versteht man in der Regel grenzflächenaktive Verbindungen, welche die Grenzflächenspannung zwischen zwei Phasen herabsetzen. Sie ermöglichen somit beispielsweise die bessere Benetzung von festen Oberflächen mit einer Flüssigkeit durch Herabsetzung der Oberflächenspannung oder die Bildung von Dispersionen. Sie werden sehr vielfältig eingesetzt, z. B. als wasch- und reinigungsaktive Substanzen in Waschmitteln, Spülmitteln, Reinigungsmitteln und Mitteln für die Körperpflege sowie als Solubilisatoren oder Netzmittel, z. B. in der Kosmetik, der Pharmazie, der Lebensmittelindustrie, dem Materialschutz, dem Pflanzenschutz, etc.

Als Emulgatoren bezeichnet man in der Regel grenzflächenaktive Verbindungen, die dazu dienen, zwei nicht miteinander mischbare Flüssigkeiten, wie z. B. Wasser und einen Ölkörper, zu einer Emulsion zu vermischen und diese gegen Entmischung zu stabilisieren.

Aufgrund der stetigen Entwicklung verschiedener neuer Produkte und Anwendungsbereiche gibt es einen ständigen Bedarf an neuen grenzflächenaktiven Verbindungen. Dabei wird eine Vielzahl von gleichzeitig zu erfüllenden Anforderungen an die neuen Verbindungen gestellt. Neben materialspezifischen Eigenschaften spielen immer öfter auch Fragen nach den Rohstoffquellen eine bedeutende Rolle. Hier wird von den Verbrauchern Produkten, die teilweise oder vollständig auf nachwachsenden Rohstoffen basieren, häufig der Vorzug gegeben. Gleichzeitig erfordert der Markt einfache und effiziente Synthesen sowie kostengünstige Ausgangsstoffe. Trotz dieser ökologischen und wirtschaftlichen Erfordernisse sollen die grenzflächenaktiven Verbindungen nach wie vor vorteilhafte Anwendungseigenschaften hinsichtlich einer Vielzahl von Eigenschaften zeigen.

Sowohl Zucker bzw. Zuckersäuren als auch Alkohole aus biogenen Quellen sind grundsätzlich geeignete Ausgangsprodukte, um neue Ester aus nachwachsenden Rohstoffen bereitzustellen. Daher sind neue grenzflächenaktive Verbindungen aus diesen Komponenten von besonderem Interesse. Der Begriff Zuckersäuren bezeichnet dabei im breiten Sinne Polyhydroxycarbonsäuren, die durch Oxidation von einfachen Zuckern (Monosacchariden) entstehen. Dabei unterscheidet man die durch Oxidation nur der Aldehydgruppe erhältlichen Aldonsäuren, die durch Oxidation der primären Alkoholgruppe erhältlichen Uronsäuren und die bei energischerer Oxidation erhältlichen Aldarsäuren, die zwei Carboxygruppen tragen (= Zuckerdisäuren). Dabei sollen die zu den Zuckersäureestern führenden Syntheserouten einfach und wirtschaftlich sein.

Es ist bekannt, dass sich bestimmte Zuckerdisäuren mit geeigneter Molekülstruktur zu Mono- und gegebenenfalls Dilactonen cyclisieren lassen.

Die WO 2006/005070 und WO 2006/005071 beschreiben Verfahren zur Herstellung von Aldonolactonen, Aldarolactonen und Aldarodilactonen. Als mögliche Einsatzbereiche für diese Lactone werden ganz pauschal die Verwendung als Zwischenprodukt für die Synthese, als chirales Ausgangsmaterial, als Enzyminhibitoren und als Monomere zur Synthese von Polymeren genannt.

Gehret, Chenault et al. beschreiben in J. Org. Chem. 2009, 74, 8373-8376 die Synthese von Glucarodilacton im Kilogramm-Maßstab. Dazu wird Calcium-D-glucarat in wässriger Acetonlösung angesäuert, Methylisobutylketon zugegeben und Aceton und Wasser durch azeotrope Destillation entfernt.

Es sind schon einige Diester von C6-Zuckerdisäuren, insbesondere von 2,3,4,5-Tetrahydroxyhexandisäuren beschrieben.

Die EP 526301 A1 beschreibt die Synthese von Galactarsäurediestern mit C₁-C₂₂-Alkyl- oder Alkenylresten aus den freien Disäuren. Beschrieben ist weiterhin die Herstellung von längerkettigen Alkyl- oder Alkenylestern durch Umesterung von den entsprechenden Niederalkylestern. In Abhängigkeit von den Reaktionsbedingungen resultieren dabei auch die 1,4-Monolactone.

Kiely et al. beschreiben im Journal of Carbohydrate Chemistry 2009, 28, 348-368 die Synthese von Poly(galactaramiden) aus gegebenenfalls substituierten Alkylendiammoniumgalactaraten. Die Diammoniumsalze werden mit methanolischer HCl in 1:1-Mischungen von Dimethylgalactarat und Alkylendiammoniumdichlorid überführt und dieses Diester/Disalz-Gemisch anschließend polymerisiert.

Amslinger et al. beschreiben in Tetrahedron 2004, 60, 11565-11569 die Synthese von Dimethylgalactarat aus Galactarsäure durch Umsetzung mit Schwefelsäure in Methanol sowie die weitere Umsetzung des Diesters zum Schützen der freien Hydroxygruppen.

Hirasaka und Umemoto beschreiben im Chem. Pharm. Bull. 1965, 13, 325-329 Untersuchungen zur Struktur des D-Glucarsäuredilactons. Die Herstellung des D-Glucarsäuredilactons erfolgt unter anderem in mehreren Stufen ausgehend vom D-Glucarsäuredimethylester, der seinerseits durch Umsetzung von Monokaliumglucarat mit Methanol in Gegenwart eines sauren Ionenaustauschers erhalten wird.

Des Weiteren sind Glucar- und Mannarsäurediester mit freien Hydroxygruppen im Zuckergrundgerüst beschrieben, unter anderem Di-n-propylglucarat, Di-n-butylglucarat, Bis-(2-methylpropyl)glucarat in Izvestiya Vysshikh Uchebnykh Zavedenii, Khimiya i Khimicheskaya Tekhnologiya 1980, 23 (11), 1409-1412 und Di-n-hexylglucarat in der US 2595636.

Kiely et al. beschreiben in J. Am. Chem. Soc. 1994, 116, 571-578 die Synthese von hydroxylierten Polyamiden aus Estern der Glucarsäure. Die Herstellung des Dimethylesters und des Diethylesters der Glucarsäure erfolgt in einer zweistufigen Reaktion, wobei die freie Disäure zunächst unter Wasserentfernung durch Gefriertrocknen in ein Gemisch des 1,4-Monolactons und des 6,3-Monolactons überführt und dieses anschließend in alkoholischer HCl zum Diester umgesetzt wird. An anderer Stelle ist beschrieben, dass die Glucaromonomethylestermonolactone und Glucaromonoethylestermonolactone im Gleichgewicht mit dem Dilacton vorliegen können.

Carpenter, Kiely et al. beschreiben in Carbohydrate Research, 2013, 376, 29-36 unter anderem die Synthese von Mannarodilacton durch Salpetersäure-Oxidation von Mannarsäure. Im Weiteren wird das Dilacton mit HCl-saurem Methanol und Acetylchlorid zum Monomethylester-Monolacton sowie zum Dimethylester der Mannarsäure umgesetzt.

Die direkte Synthese von Dialkylestern von Zuckersäuren aus Dilactonen von Zuckersäuren war bisher lediglich für die Methylester der Mannarsäure beschrieben, wobei die entsprechende Reaktion mit HCl saurem Methanol durchgeführt wird. Diese Reaktion hat den gravierenden Nachteil, dass korrosives und giftiges HCl-Gas eingesetzt werden muss. Ein weiterer Nachteil liegt in der nicht ausreichenden Löslichkeit des HCl-Gases in höheren Alkoholen als Methanol und/oder Ethanol, wodurch Ester höherer Alkohole der 2,3,4,5- Tetrahydroxyhexansäure nicht auf diese Weise zugänglich sind.

Der vorliegenden Erfindung liegt die Aufgabe zugrunde, 2,3,4,5-Tetrahydroxyhexandisäureester durch eine einfache Syntheseroute bereitzustellen. Die Synthese soll unter milden Bedingungen ablaufen; mögliche gefährliche oder in ihrer technischen Handhabung aufwändige Reaktionskomponenten sowie die Bildung unerwünschter Nebenprodukte sollen vermieden werden. Die neuen 2,3,4,5-Tetrahydroxyhexandisäureester sollen speziell geeignet sein, ein komplexes Anforderungsspektrum abzudecken, wie es eingangs beschrieben ist.

Überraschenderweise wurde nun gefunden, dass diese Aufgabe durch die Synthese von 2,3,4,5-Tetrahydroxyhexandisäureestern aus Dilactonen von 2,3,4,5-Tetrahydroxyhexandisäure-haltigen Ausgangsstoffen durch Umsetzung mit Alkoholen gelöst wird. Dabei reagiert das Dilacton mit der Alkoholkomponente unter Ringöffnung zu Diestern und gegebenenfalls zusätzlich zu Monoestern der 2,3,4,5-Tetrahydroxyhexandisäure. Dabei ist es vorteilhafterweise nicht erforderlich, die Lactone der 2,3,4,5-Tetrahydroxyhexandisäure zunächst einer Ringöffnung in wässrigem Medium und anschließend einer klassischen Veresterung unter dehydratisierenden Bedingungen zu unterziehen. Es hat sich weiterhin als vorteilhaft erwiesen, die Umsetzung in Gegenwart eines Katalysators durchzuführen, wobei vorzugsweise keine Protonensäuren eingesetzt werden. Insbesondere wird für die Veresterung in Schritt b) keine Mineralsäure eingesetzt. Das Dilacton würde in Gegenwart mit einer starken Mineralsäure, die zudem noch entwässernd wirkt, in einer heftigen Reaktion Wasser abspalten und sich zersetzen, ohne mit dem Alkohol einen Ester zu bilden. Das erfindungsgemäße Verfahren ermöglicht eine Umsetzung bei milden Temperaturen, wodurch die thermische Zersetzung der Lactone der 2,3,4,5-Tetrahydroxyhexandisäure vermieden wird, wie sie bei Veresterungen aus dem Stand der Technik problematisch ist. Ein weiterer Vorteil gegenüber einer klassischen Veresterung aus freier Säure und Alkohol ist, dass bei der Ringöffnung des Dilactons kein Wasser entsteht. Des Weiteren zeichnet sich das erfindungsgemäße Verfahren durch seine hohe Atomeffizienz aus.

### ZUSAMMENFASSUNG DER ERFINDUNG

Gegenstand der Erfindung ist ein Verfahren zur Herstellung von 2,3,4,5-Tetrahydroxyhexandisäureestern umfassend wenigstens eine Verbindung der allgemeinen Formel (1), 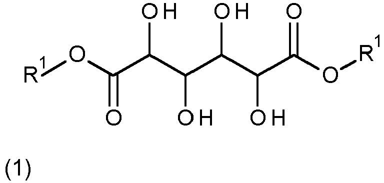 worin die Reste R¹ unabhängig voneinander für C₁-C₂₄-Alkyl, C₂-C₂₄-Alkenyl mit 1, 2, 3 oder mehr als 3 C-C-Doppelbindungen, oder für eine Gruppe der Formel -(R²⁻O)ₙ-R³ stehen, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
bei dem man
a) eine Verbindung der Formel (2) 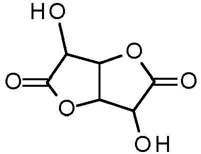 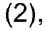 die ausgewählt ist unter Verbindungen der Formeln (2.a), (2.b) und (2.c). 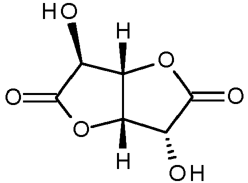 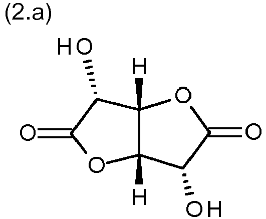 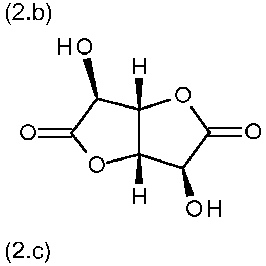 bereitstellt, und
b) die in Schritt a) bereitgestellte Verbindung der Formel (2) mit wenigstens einer Verbindung der allgemeinen Formel (3),

   R¹-OH (3)

   worin R¹ die oben angegebene Bedeutung besitzt,
in Gegenwart wenigstens eines Katalysators umsetzt, welcher ausgewählt ist unter Verbindungen, die wenigstens ein Lewis-saures, hartes Kation und/oder wenigstens ein hartes Anion aufweisen.

Ein weiterer Gegenstand der Erfindung sind Verbindungen der allgemeinen Formel (1), welche bevorzugt ausgewählt sind unter

Verbindungen der Formel (1.a), 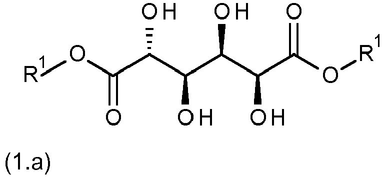
worin einer der Reste R¹ für C₁-C₂₄-Alkyl, C₂-C₂₄-Alkenyl mit 1, 2, 3 oder mehr als 3 C-C-Doppelbindungen, oder für eine Gruppe der Formel -(R²⁻O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₈-C₂₄-Alkyl, C₂-C₂₄-Alkenyl mit 1, 2, 3 oder mehr als 3 C-C-Doppelbindungen, oder für eine Gruppe der Formel -(R²⁻O)ₙ-R³ steht, worin jedes R² ausgewählt ist unter C₁-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht;

Verbindungen der Formel (1.b), 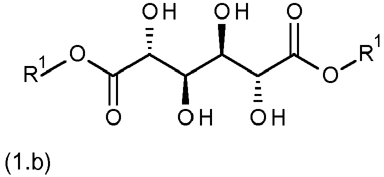
worin einer der Reste R¹ für C₁-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₃-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht; und

Verbindungen der Formel (1.c), 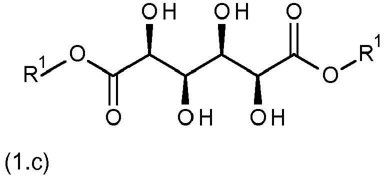
worin einer der Reste R¹ für C₁-C₂₄-Alkyl, C₂-C₂₄-Alkenyl mit 1, 2, 3 oder mehr als 3 C-C-Doppelbindungen oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₁-C₂₄-Alkyl, C₂-C₂₄-Alkenyl mit 1, 2, 3 oder mehr als 3 C-C-Doppelbindungen oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht.

Ein weiterer Gegenstand der Erfindung sind Wasch-, Reinigungs- oder Geschirrspülmittel oder Klarspüler, enthaltend wenigstens eine Verbindung der allgemeinen Formel (1), wie in Anspruch 9 definiert, und wenigstens ein davon verschiedenes Tensid.

Ein weiterer Gegenstand der Erfindung sind kosmetische oder pharmazeutische Zusammensetzungen, enthaltend wenigstens eine Verbindung der allgemeinen Formel (1), wie in Anspruch 9 definiert, und wenigstens einen davon verschiedenen kosmetischen oder pharmazeutischen Wirkstoff und/oder Hilfsstoff.

Ein weiterer Gegenstand der Erfindung ist die Verwendung wenigstens einer Verbindung der allgemeinen Formel (1), wie in Anspruch 9 definiert, als grenzflächenaktive Verbindung, bevorzugt als Tensid, Emulgator oder Schaumbildner.

Ein weiterer Gegenstand der Erfindung ist die Verwendung wenigstens einer Verbindung der allgemeinen Formel (1), wie in Anspruch 9 definiert, als grenzflächenaktive Verbindung in kosmetischen Mitteln, in pharmazeutischen Mitteln, in Wasch- und Reinigungsmitteln, in Geschirrspülmitteln und in Klarspülern, in Hygieneprodukten, in Pflanzenschutzmitteln, in Lacken, Beschichtungsmitteln, Klebstoffen, Lederbehandlungs- oder Textilbehandlungsmitteln, sowie bei der Erschließung und/oder Ausbeutung unterirdischer Erdöl- und/oder Erdgaslagerstätten.

### BESCHREIBUNG DER ERFINDUNG

Im Rahmen der Erfindung bezeichnet der Begriff "2,3,4,5-Tetrahydroxyhexandisäureester" sowohl Diester der Aldarsäuren der allgemeinen Formel (1) als auch Monoestermonolactone der allgemeinen Formel (1').
Im Rahmen der Erfindung bezeichnet der Begriff "2,3,4,5-Tetrahydroxyhexandisäureester" eine Verbindung oder mehrere Verbindungen der allgemeinen Formeln (1) und (1') sowie alle Konfigurationsisomere der Verbindungen (1) und (1'), wie die Verbindungen (1a), (1b) und (1c). Umfasst sind zusätzlich alle Stereoisomere.

Im Rahmen der Erfindung bezeichnet der Begriff "2,3,4,5-Tetrahydroxyhexandisäurediester" eine Verbindung oder mehrere Verbindungen der allgemeinen Formel (1) sowie alle Konfigurationsisomere der Verbindungen (1), wie die Verbindungen (1a), (1b) und (1c). Umfasst sind zusätzlich alle Stereoisomere.

Im Rahmen der Erfindung bezeichnet der Begriff "Dilacton der 2,3,4,5-Tetrahydroxyhexandisäure" eine Verbindung oder mehrere Verbindungen der allgemeinen Formel (2) sowie alle Konfigurationsisomere der Verbindungen (2), wie die Verbindungen (2a), (2b) und (2c). Umfasst sind zusätzlich alle Stereoisomere.

Die Begriffe "Rheologiemodifizierer" und "Modifizierung rheologischer Eigenschaften" werden im Rahmen der vorliegenden Erfindung weit verstanden. Die entsprechend eingesetzten Verbindungen der Formel (1) und (1') eignen sich im Allgemeinen zur Verdickung der Konsistenz von flüssigen Zusammensetzungen in einem weiten Bereich. Je nach Grundkonsistenz der flüssigen Zusammensetzungen können in Abhängigkeit von der Einsatzmenge der Verbindungen der allgemeinen Formel (1) und (1') in der Regel Fließeigenschaften von dünnflüssig bis hin zu fest (im Sinne von "nicht mehr fließend") erzielt werden. Unter "Modifizierung rheologischer Eigenschaften" wird daher unter anderem die Erhöhung der Viskosität von Flüssigkeiten, die Verbesserung der Thixotropie-Eigenschaften von Flüssigkeiten und Gelen, die Verfestigung von Gelen und Wachsen, etc. verstanden. Die Verbindungen der Formel (1) und (1') eignen sich speziell zur Modifizierung der rheologischen Eigenschaften wässriger Zusammensetzungen.

Geeignete C₁-C₂₄-Alkylgruppen, C₈-C₂₄-Alkylgruppen, C₈-C₁₀-Alkylgruppen, C₁₂-C₁₄-Alkylgruppen und C₁₆-C₂₄-Alkylgruppen sind jeweils lineare und verzweigte Alkylgruppen.

Geeignete C₂-C₂₄-Alkenylgruppen, C₃-C₂₄-Alkenylgruppen, C₈-C₂₄-Alkenylgruppen, C₈-C₁₀-Alkenylgruppen, C₁₂-C₁₄-Alkenylgruppen und C₁₆-C₂₄-Alkenylgruppen sind jeweils lineare und verzweigte Alkenylgruppen mit jeweils mit 1, 2, 3 oder mehr als 3 C-C-Doppelbindungen, z. B. 4, 5 oder 6 Doppelbindungen.

Geeignete C₂-C₁₀-Alkylengruppen und C₁-C₄-Alkylengruppen sind jeweils lineare und verzweigte Alkenylgruppen.
In einer bevorzugten Ausführungsform handelt es sich um überwiegend lineare Alkylreste, wie sie auch in natürlichen oder synthetischen Fettalkoholen sowie Oxoalkoholen vorkommen, bzw. um überwiegend lineare Alkenylreste, wie sie auch in natürlichen oder synthetischen Fettalkoholen sowie Oxoalkoholen vorkommen, die einfach, zweifach, dreifach, vierfach, fünffach oder sechsfach ungesättigt sein können.

Wenn der Rest R¹ für Alkyl oder Alkenyl steht, so stammen diese Reste vorzugsweise aus natürlichen Rohstoffen, besonders bevorzugt aus einem nachwachsenden Rohstoff.

Weiterhin bevorzugt stammen die zur Herstellung der Verbindungen der allgemeinen Formel (1) eingesetzten Ausgangsmaterialien zumindest teilweise aus einer erneuerbaren, natürlichen und/oder nachwachsenden Quelle oder erfolgt deren Herstellung aus natürlichen und/oder nachwachsenden Rohstoffen.

Unter erneuerbaren Quellen werden im Sinne der Erfindung natürliche (biogene) und/oder nachwachsende Quellen verstanden und nicht fossile Quellen, wie Erdöl, Erdgas oder Kohle. Aus erneuerbaren Quellen gewonnene Verbindungen (natürliche und/oder nachwachsende Rohstoffe) weisen ein anderes ¹⁴C-zu-¹²C-Isotopenverhältnis als aus fossilen Quellen, wie Erdöl, gewonnene Verbindungen auf. Die in dem erfindungsgemäßen Verfahren eingesetzten Verbindungen der allgemeinen Formeln (2) und/oder (3) weisen somit vorzugsweise ein ¹⁴C-zu-¹²C-Isotopenverhältnis im Bereich von 0,5×10⁻¹² bis 5×10⁻12 auf.

Die Reste R¹ sind unabhängig voneinander ausgewählt unter C₁-C₂₄-Alkyl, C₂-C₂₄-Alkenyl mit 1, 2, 3 oder mehr als 3 C-C-Doppelbindungen und einer Gruppe der Formel -(R²-O)ₙ-R³, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht.

Bevorzugt sind in den Verbindungen der Formel (1) die Reste R¹ unabhängig voneinander ausgewählt unter linearen oder verzweigten C₈-C₂₄-Alkylgruppen.

Besonders bevorzugt sind in den Verbindungen der Formel (1) die Reste R¹ unabhängig voneinander ausgewählt unter linearen oder verzweigten C₈-C₁₀-Alkylgruppen. Diese eignen sich vorteilhaft als Klarspüladditive.

Weiterhin besonders bevorzugt sind in den Verbindungen der Formel (1) die Reste R¹ unabhängig voneinander ausgewählt unter linearen oder verzweigten C₁₂-C₁₄-Alkylgruppen. Diese eignen sich vorteilhaft als Tenside.

Weiterhin besonders bevorzugt sind in den Verbindungen der Formel (1) die Reste R¹ unabhängig voneinander ausgewählt unter linearen oder verzweigten C₁₆-C₂₄-Alkylgruppen. Diese eignen sich vorteilhaft als Verdicker und Rheologiemodifizierer.

Weiterhin bevorzugt sind in den Verbindungen der Formel (1) die Reste R¹ unabhängig voneinander ausgewählt unter linearen oder verzweigten C₃-C₂₄-Alkenylgruppen mit 1, 2, 3 oder 4 C-C-Doppelbindungen.

Weiterhin besonders bevorzugt sind in den Verbindungen der Formel (1) die Reste R¹ unabhängig voneinander ausgewählt unter linearen oder verzweigten C₈-C₂₄-Alkenylgruppen mit 1, 2, 3 oder 4 C-C-Doppelbindungen.

Weiterhin ganz besonders bevorzugt sind in den Verbindungen der Formel (1) die Reste R¹ unabhängig voneinander ausgewählt unter linearen oder verzweigten C₈-C₁₀-Alkenylgruppen mit 1, 2, 3 oder 4 C-C-Doppelbindungen. Diese eignen sich vorteilhaft als Klarspüladditive.

Weiterhin ganz besonders bevorzugt sind in den Verbindungen der Formel (1) die Reste R¹ unabhängig voneinander ausgewählt unter linearen oder verzweigten C₁₂-C₁₄-Alkenylgruppen mit 1, 2, 3 oder 4 C-C-Doppelbindungen. Diese eignen sich vorteilhaft als Tenside.

Weiterhin ganz besonders bevorzugt sind in den Verbindungen der Formel (1) die Reste R¹ unabhängig voneinander ausgewählt unter linearen oder verzweigten C₁₆-C₂₄-Alkenylgruppen mit 1, 2, 3 oder 4 C-C-Doppelbindungen. Diese eignen sich vorteilhaft als Verdicker und Rheologiemodifizierer.

Weiterhin bevorzugt sind in den Verbindungen der Formel (1) die Reste R¹ unabhängig voneinander ausgewählt unter einer Gruppe der Formel -(R²-O)ₙ-R³.

Dann ist R² vorzugsweise ausgewählt unter C₂-C₄-Alkylen. R³ steht dann vorzugsweise für C₈-C₂₄-Alkyl. n steht dann vorzugsweise für natürliche Zahl von 1 bis 500.

Insbesondere ist R² ausgewählt ist unter C₂-C₄-Alkylen, R³ steht für C₈-C₂₄-Alkyl oder C₈-C₂₄-Alkenyl mit 1, 2, 3 oder mehr als 3 C-C-Doppelbindungen und n steht für eine natürliche Zahl von 1 bis 500.

Bevorzugt sind in den Verbindungen der Formel (1') die Reste R¹ unabhängig voneinander ausgewählt unter linearen oder verzweigten C₈-C₂₄-Alkylgruppen.

Besonders bevorzugt sind in den Verbindungen der Formel (1') die Reste R¹ unabhängig voneinander ausgewählt unter linearen oder verzweigten C₈-C₁₀-Alkylgruppen. Diese eignen sich vorteilhaft als Klarspüladditive.

Weiterhin besonders bevorzugt sind in den Verbindungen der Formel (1') die Reste R¹ unabhängig voneinander ausgewählt unter linearen oder verzweigten C₁₂-C₁₄-Alkylgruppen. Diese eignen sich vorteilhaft als Tenside.

Weiterhin besonders bevorzugt sind in den Verbindungen der Formel (1') die Reste R¹ unabhängig voneinander ausgewählt unter linearen oder verzweigten C₁₆-C₂₄-Alkylgruppen. Diese eignen sich vorteilhaft als Verdicker und Rheologiemodifizierer,

Weiterhin bevorzugt sind in den Verbindungen der Formel (1') die Reste R¹ unabhängig voneinander ausgewählt unter linearen oder verzweigten C₃-C₂₄-Alkenylgruppen mit 1, 2, 3 oder 4 C-C-Doppelbindungen.

Weiterhin besonders bevorzugt sind in den Verbindungen der Formel (1') die Reste R¹ unabhängig voneinander ausgewählt unter linearen oder verzweigten C₈-C₂₄-Alkenylgruppen mit 1, 2, 3 oder 4 C-C-Doppelbindungen.

Weiterhin ganz besonders bevorzugt sind in den Verbindungen der Formel (1') die Reste R¹ unabhängig voneinander ausgewählt unter linearen oder verzweigten C₈-C₁₀-Alkenylgruppen mit 1, 2, 3 oder 4 C-C-Doppelbindungen. Diese eignen sich vorteilhaft als Klarspüladditive.

Weiterhin ganz besonders bevorzugt sind in den Verbindungen der Formel (1') die Reste R¹ unabhängig voneinander ausgewählt unter linearen oder verzweigten C₁₂-C₁₄-Alkenylgruppen mit 1, 2, 3 oder 4 C-C-Doppelbindungen. Diese eignen sich vorteilhaft als Tenside.

Weiterhin ganz besonders bevorzugt sind in den Verbindungen der Formel (1') die Reste R¹ unabhängig voneinander ausgewählt unter linearen oder verzweigten C₁₆-C₂₄-Alkenylgruppen mit 1, 2, 3 oder 4 C-C-Doppelbindungen. Diese eignen sich vorteilhaft als Verdicker und Rheologiemodifizierer.

Weiterhin bevorzugt sind in den Verbindungen der Formel (1') die Reste R¹ unabhängig voneinander ausgewählt unter einer Gruppe der Formel -(R²-O)ₙ-R³.

Dann ist R² vorzugsweise ausgewählt unter C₂-C₄-Alkylen. R³ steht dann vorzugsweise für C₈-C₂₄-Alkyl. n steht dann vorzugsweise für natürliche Zahl von 1 bis 500.

Insbesondere ist R² ausgewählt ist unter C₂-C₄-Alkylen, R³ steht für C₈-C₂₄-Alkyl oder C₈-C₂₄-Alkenyl mit 1, 2, 3 oder mehr als 3 C-C-Doppelbindungen und n steht für eine natürliche Zahl von 1 bis 500 steht.

Wenn R¹ für C₈-C₁₀-Alkyl steht, so ist dieses linear oder verzweigt und ist vorzugsweise ausgewählt unter n-Octyl, 2-Ethylhexyl, n-Nonyl, n-Decyl und deren Konstitutionsisomeren.

Wenn R¹ für C₁₂-C₁₄-Alkyl steht, so ist dieses linear oder verzweigt und ist vorzugsweise ausgewählt unter n-Dodecyl, n-Tridecyl, Isotridecyl, n-Tetradecyl und deren Konstitutionsisomeren.

Wenn R¹ für C₁₆-C₂₄-Alkyl steht, so ist dieses linear oder verzweigt und ist vorzugsweise ausgewählt unter n-Hexadecyl, n-Heptadecyl, n-Octadecyl, Isostearyl, n-Nonadecyl, Arachinyl, Behenyl, Lignoceryl und deren Konstitutionsisomeren.

Wenn R¹ für C₈-C₂₄-Alkyl steht, so ist dieses linear oder verzweigt und ist vorzugsweise ausgewählt unter den zuvor aufgeführten C₈-C₁₀-Alkylresten, den zuvor aufgeführten C₁₂-C₁₄-Alkylresten, den zuvor aufgeführten C₁₆-C₂₄-Alkylresten sowie unter n-Undecyl, n-Pentadecyl und deren Konstitutionsisomeren.

Wenn R¹ für C₁-C₂₄-Alkyl steht, so ist dieses linear oder verzweigt und ist vorzugsweise ausgewählt unter den zuvor aufgeführten C₈-C₂₄-Alkylresten sowie unter Methyl, Ethyl, n-Propyl, n-Butyl, n-Pentyl, n-Hexyl, n-Heptyl und deren Konstitutionsisomeren.

Wenn R¹ für C₈-C₁₀-Alkenyl steht, so ist dieses linear oder verzweigt und ist vorzugsweise ausgewählt unter n-Octenyl, n-Octadienyl, n-Octatrienyl, n-Nonenyl, n-Nonadienyl, n-Nonatrienyl, n-Decenyl, n-Decadienyl, n-Decatrienyl und deren Konstitutionsisomeren.

Wenn R¹ für C₁₂-C₁₄-Alkenyl steht, so ist dieses linear oder verzweigt und ist vorzugsweise ausgewählt unter n-Dodecenyl, n-Dodecadienyl, n-Dodecatrienyl, n-Tridecenyl, n-Tridecadienyl, n-Tridecatrienyl, n-Tetradecenyl, n-Tetradecadienyl n-Tetradecatrienyl und deren Konstitutionsisomeren.

Wenn R¹ für C₁₆-C₂₄-Alkenyl steht, so ist dieses linear oder verzweigt und ist vorzugsweise ausgewählt unter n-Hexadecenyl, n-Hexadecadienyl, n-Hexadecatrienyl, n-Heptadecenyl, n-Heptadecadienyl, n-Heptadecatrienyl, n-Octadecenyl, n-Octadecadienyl, n-Octadecatrienyl, n-Nonadecenyl, n-Nonadecadienyl, n-Nonadecatrienyl, n-Eicosenyl, n-Eicosadienyl, n-Eicosatrienyl, n-Heneicosenyl, n-Heneicosadienyl, n-Heneicosatrienyl, n-Docosenyl, n-Docosadienyl, n-Docosatrienyl, n-Tricosenyl, n-Tricosadienyl, n-Tricosatrienyl, n-Tetracosenyl, n-Tetracosadienyl, n-Tetracosatrienyl,Oleyl, Linolenyl und deren Konstitutionsisomeren.

Wenn R¹ für C₈-C₂₄-Alkenyl steht, so ist dieses linear oder verzweigt und ist vorzugsweise ausgewählt unter den zuvor aufgeführten C₈-C₁₀-Alkenylresten, den zuvor aufgeführten C₁₂-C₁₄-Alkenylresten, den zuvor aufgeführten C₁₆-C₂₄-Alkenylresten sowie unter n-Undecenyl, n-Undecadienyl, n-Undecatrienyl, n-Pentadecenyl, n-Pentadecadienyl, n-Pentadecatrienyl und deren Konstitutionsisomeren.

Wenn R¹ für C₃-C₂₄-Alkenyl steht, so ist dieses linear oder verzweigt und ist vorzugsweise ausgewählt unter den zuvor aufgeführten C₈-C₂₄-Alkenylresten sowie unter n-Propenyl, Allyl, n-Butenyl, n-Butadienyl, n-Pentenyl, n-Pentadienyl, n-Hexenyl, n-Hexadienyl, n-Heptenyl, n-Heptadienyl und deren Konstitutionsisomeren.
Wenn R¹ für C₂-C₂₄-Alkenyl steht, so ist dieses linear oder verzweigt und ist vorzugsweise ausgewählt unter den zuvor aufgeführten C₃-C₂₄-Alkenylresten sowie unter Ethenyl und Vinyl.

Wenn R² für C₂-C₄ Alkylen steht, so ist dieses linear oder verzweigt und ist vorzugsweise ausgewählt unter -CH₂CH₂-, -CH(CH₃)-, -CH₂CH₂CH₂-, -CH(CH₃)CH₂-, -CH₂CH₂CH₂CH₂-, -CH₂CH(CH₃)CH₂-, -CH(CH₃)CH(CH₃)- und deren Konstitutionsisomeren.

Wenn R² für C₂-C₁₀ Alkylen steht, so ist dieses linear oder verzweigt und ist vorzugsweise ausgewählt unter den zuvor aufgeführten C₂-C₄-Alkylenresten sowie unter -C₅H₁₀-,-C₆H₁₂-,-C₇H₁₄-,-C₈H₁₆-,-C₉H₁₈-,-C₁₀H₂₀- und deren Konstitutionsisomeren.

Wenn R³ für C₈-C₂₄-Alkyl steht, so ist dieses linear oder verzweigt und ist vorzugsweise ausgewählt unter den zuvor für R¹ aufgezählten C₈-C₂₄-Alkylresten.

Wenn R³ für C₁-C₂₄-Alkyl steht, so ist dieses linear oder verzweigt und ist vorzugsweise ausgewählt unter den zuvor für R¹ aufgezählten C₁-C₂₄-Alkylresten.

Wenn R³ für C₈-C₂₄-Alkenyl steht, so ist dieses linear oder verzweigt und ist vorzugsweise ausgewählt unter den zuvor für R¹ aufgezählten C₈-C₂₄-Alkenylresten.

Wenn R³ für C₃-C₂₄-Alkenyl steht, so ist dieses linear oder verzweigt und ist vorzugsweise ausgewählt unter den zuvor für R¹ aufgezählten C₃-C₂₄-Alkenylresten.

Wenn R³ für C₂-C₂₄-Alkenyl steht, so ist dieses linear oder verzweigt und ist vorzugsweise ausgewählt unter den zuvor für R¹ aufgezählten C₂-C₂₄-Alkenylresten.

Die Reste R¹, die für C₁-C₂₄-Alkyl, C₈-C₂₄-Alkyl, C₈-C₁₀-Alkyl, C₁₂-C₁₄-Alkyl, C₁₆-C₂₄-Alkyl, C₂-C₂₄-Alkenyl, C₃-C₂₄-Alkenyl, C₈-C₂₄-Alkenyl, C₈-C₁₀-Alkenyl, C₁₂-C₁₄-Alkenyl und C₁₆-C₂₄-Alkenyl stehen, können sich von den entsprechenden Alkoholen der allgemeinen Formel R¹-OH durch formale Abspaltung der OH-Gruppe ableiten. Die Reste R¹ können sich von reinen Alkoholen oder von Alkoholgemischen ableiten. Bevorzugt handelt es sich um großtechnische verfügbare Alkohole oder Alkoholgemische. In einer bevorzugten Ausführungsform sind die Reste R¹ dann unabhängig voneinander ausgewählt unter überwiegend linearen Alkyl-, Alkenyl-, Alkadienyl- und Alkatrienylresten, wie sie in natürlichen oder synthetischen Fettalkoholen vorkommen. In einer geeigneten Ausführungsform sind die Reste R¹ unabhängig voneinander abgeleitet von Fettalkoholen, die auf technischen Fettalkoholmischungen basieren. Bevorzugt sind die Reste R¹ unabhängig voneinander abgeleitet von natürlich vorkommenden Fettalkoholen und Fettalkoholgemischen.

Bevorzugte Verbindungen der allgemeinen Formel (3) sind 1-Hexanol, 1-Heptanol, 1-Octanol, 1-Nonanol, 1-Decanol, 1-Undecanol, 1-Dodecanol (Laurylalkohol), 1-Tridecanol, 1-Tetradecanol (Myristylalkohol), 1-Pentadecanol, 1-Hexadecanol (Cetylalkohol oder Palmitylalkohol), 1-Heptadecanol (Margarylalkohol), 1-Octadecanol (Stearylalkohol), Isostearylalkohol, 1-Eicosanol (Arachidylalkohol), 1-Docosanol (Behenylalkohol), 1-Tetracosanol (Lignocerylalkohol), Allylalkohol, cis-9-Hexadecen-1-ol, cis-9-Octadecen-1-ol (Oleylalkohol), trans-9-Octadecen-1-ol (Elaidylalkohol), cis-11-Octadecen-1-ol und 6,9,12-Octadecatrien-1-ol (γ-Linolenylalkohol).

In einer speziellen Ausführungsform wird als Komponente (3) eine Mischung aus wenigstens zwei verschiedenen Alkoholen eingesetzt. Bevorzugt wird dann eine aus natürlichen Quellen oder technischen Prozessen zur Verfügung stehende Alkoholmischung eingesetzt. Besonders bevorzugt wird als Komponente (3) eine aus nachwachsenden Rohstoffen erhaltene Mischung aus wenigstens zwei verschiedenen Alkoholen eingesetzt. Eine als Komponente (3) bevorzugte Alkoholmischung umfasst 1-Dodecanol und 1-Tetradecanol. Derartige Mischungen sind unter der Bezeichnung Lorol® spezial von der BASF SE erhältlich. Eine weitere als Komponente (3) bevorzugte Alkoholmischung ist ein aus pflanzlichen Rohstoffen erhaltenes Alkoholgemisch, welches als Hauptkomponente Oleylalkohol enthält. Geeignet ist z. B. ein Gemisch, enthaltend, jeweils bezogen auf das Gesamtgewicht der Alkoholmischung, 0 bis 2 Gew.-% C₁₄-Alkohole, 2 bis 10 Gew.-% C₁₆-Alkohole, 90 bis 98 Gew.-% Oleylalkohol und 0 bis 3 Gew.-% höhere Alkohole als C₁₈-Alkohole. Eine derartige Mischung ist unter der Bezeichnung HD Ocenol® 90/95 von der BASF SE erhältlich.

Wenn R¹ für eine Gruppe der Formel -(R²-O)ₙ-R³ steht, kann R² in jeder Wiederholungseinheit unabhängig voneinander für lineares oder verzweigtes C₂-C₁₀-Alkylen stehen, R³ für lineares oder verzweigtes C₁-C₂₄-Alkyl oder C₂-C₂₄-Alkenyl mit 1, 2, 3 oder mehr als 3 C-C-Doppelbindungen stehen und n für eine natürliche Zahl von 1 bis 1000 stehen.

Erfindungsgemäß steht n für eine natürliche Zahl zwischen 1 und 1000. Bevorzugt steht n für eine natürliche Zahl zwischen 1 und 500, besonders bevorzugt für eine natürliche Zahl zwischen 2 und 100 und ganz besonders bevorzugt für eine natürliche Zahl zwischen 4 und 20.

Sofern in den Gruppen der Formel -(R²-O)ₙ-R³ die Variable n für wenigstens 2 steht, können die Reste R² der einzelnen Wiederholungseinheiten gleiche oder verschiedene Bedeutungen aufweisen. Grundsätzlich ist die Reihenfolge der Alkylenoxideinheiten (R²-O) beliebig.

Der Ausdruck "C₂-C₁₀-Alkylen" (oder C₂-C₁₀-Alkandiyl) bezeichnet einen Alkylrest mit 2 bis 10 Kohlenstoffatomen, wobei ein Wasserstoffatom an einer beliebigen Stelle des Alkylrestes durch eine weitere Bindungsstelle ersetzt ist, so dass eine bivalente Gruppe gebildet wird. Geeignete Beispiele sind oben genannt.

Der Ausdruck "C₂-C₄-Alkylen" (oder C₂-C₄-Alkandiyl) bezeichnet einen Alkylrest mit 2 bis 4 Kohlenstoffatomen, wobei ein Wasserstoffatom an einer beliebigen Stelle des Alkylrestes durch eine weitere Bindungsstelle ersetzt ist, so dass eine bivalente Gruppe gebildet wird. Geeignete Beispiele sind oben genannt.

In einer Ausführungsform steht R³ für Methyl, Ethyl, n-Propyl, Isopropyl, n-Butyl, sec.-Butyl, n-Pentyl, n-Hexyl, n-Heptyl, n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arachinyl, Behenyl, Lignocerenyl, Palmitoleinyl, Oleyl, Linolyl, Linolenyl, Stearyl oder Lauryl.

In einer geeigneten Ausführungsform kann in den Gruppen der Formel -(R²-O)ₙ-R³ der Rest R³ auch für ein Gemisch verschiedener Reste stehen. Beispielsweise kann R³ für Cetearyl stehen, d. h. ein Gemisch aus Cetyl und Stearyl.

Geeignete (Poly-)Etherole HO-(R²-O)ₙ-R³ zur Herstellung der Verbindungen (1) oder (1') können leicht durch Umsetzung von Ethylenoxid, 1,2-Propylenoxid, 1,2-Butylenoxid, 2,3-Butylenoxid, THF und/oder Epichlorhydrin mit einem kurzkettigen Alkohol R³-OH als Startermolekül hergestellt werden. Die Alkylenoxide können einzeln, alternierend nacheinander oder als Mischung eingesetzt werden.

Die Herstellung von Verbindungen der allgemeinen Formel (2) für den Einsatz in Schritt a) ist dem Fachmann bekannt, beispielsweise aus den eingangs genannten Dokumenten. Insbesondere sind in den Dokumenten WO 06005070 A1 und WO 06005071 A1 Synthesen von Glucarodilacton (Verbindung 2.a), Mannarodilacton (Verbindung 2.b) und Idarodilacton (Verbindung 2.c) beschrieben. Auf die Offenbarung dieser Dokumente wird in vollem Umfang Bezug genommen.

In einer bevorzugten Ausführungsform des erfindungsgemäßen Verfahrens umfassen die hergestellten 2,3,4,5-Tetrahydroxyhexandisäureester zusätzlich wenigstens eine Verbindung der allgemeinen Formel (1') 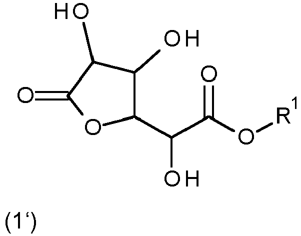 wobei R¹ eine der oben angegebenen Bedeutungen besitzt.

Bevorzugt enthalten die nach dem erfindungsgemäßen Verfahren erhaltenen 2,3,4,5-Tetrahydroxyhexandisäureester die Verbindungen der allgemeinen Formel (1') in einer Menge von 0 bis 90 Gew.-%, bevorzugt von 0,01 bis 75 Gew.-%, besonders bevorzugt von 0,1 bis 67 Gew.-% und insbesondere bevorzugt von 1 bis 55 Gew.-%, bezogen auf das Gesamtgewicht der Verbindungen (1) und (1').

Nach dem erfindungsgemäßen Verfahren ist das in Schritt a) bereitgestellte Dilacton der 2,3,4,5-Tetrahydroxyhexandisäure der allgemeinen Formel (2) ausgewählt unter Verbindungen der Formeln (2.a), (2.b) und (2.c).

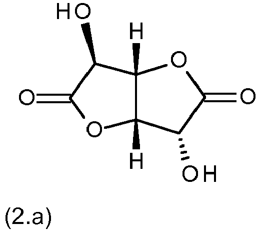

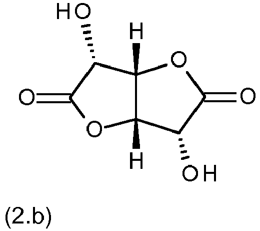

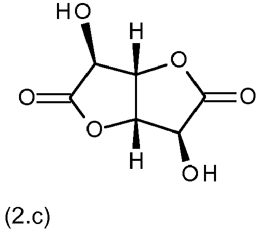

Eine besonders bevorzugte Ausführungsform ist ein Verfahren, wobei die in Schritt a) bereitgestellte Verbindung der allgemeinen Formel (2) die Verbindung der Formel (2.a) ist.

Vorzugsweise beträgt in Schritt b) des erfindungsgemäßen Verfahrens die Gesamtmenge an Verbindungen der Formel (3) bezogen auf die Menge der Verbindung (2) 0,5 bis 100 Moläquivalente, besonders bevorzugt 0,5 bis 50 Moläquivalente, insbesondere 1 bis 10 Moläquivalente und ganz besonders bevorzugt 1 bis 4 Moläquivalente.

Vorzugsweise wird die Veresterung in Schritt b) bei einer Temperatur in einem Bereich von 10 bis 150 °C, besonders bevorzugt in einem Bereich von 15 bis 90 °C und insbesondere in einem Bereich von 20 bis 60 °C, durchgeführt. Bei diesen niedrigen Temperaturen kann vorteilhafterweise eine thermische Zersetzung der Bislactone (2) und der resultierenden Verbindungen (1) vermieden werden, wie sie bei den aus dem Stand der Technik bekannten Veresterungen im stark sauren Medium und bei erhöhter Temperatur beobachtet wird.

Die Umsetzung in Schritt b) erfolgt in Gegenwart eines Katalysators.

Speziell erfolgt die Umsetzung in Schritt b) nicht in Gegenwart einer Protonensäure, also nicht in Gegenwart von H+. Insbesondere wird für die Veresterung in Schritt b) keine Mineralsäure eingesetzt. Der Begriff Mineralsäure bezeichnet dabei im Rahmen der Erfindung die starken anorganischen Säuren Salzsäure (Chlorwasserstoffsäure), Schwefelsäure, Phosphorsäure und Salpetersäure. Insbesondere wird für die Veresterung in Schritt b) keine Mineralsäure eingesetzt. Das Dilacton würde in Gegenwart mit einer starken Mineralsäure, insbesondere in Gegenwart einer stark dehydrierenden Mineralsäure, in einer heftigen Reaktion Wasser abspalten und sich zersetzen, ohne mit dem Alkohol einen Ester zu bilden.

In einer bevorzugten Ausführungsform wird das Verfahren zur Herstellung von 2,3,4,5-Tetrahydroxyhexandisäureestern in Schritt b) in Gegenwart wenigstens eines Katalysators umsetzt, welcher ausgewählt ist unter Verbindungen, die wenigstens ein Lewis-saures, hartes Kation und/oder wenigstens ein hartes Anion aufweisen.

Die Begriffe "Verbindungen mit harten Kationen" und "harte Anionen" haben für den Fachmann einen klaren Begriffsinhalt. Dieser ergibt sich aus dem Konzept der harten und weichen Säuren und Basen (HSAB-Konzept nach Pearson) wie es beispielsweise in JACS 1963, 85, 3533 beschrieben ist sowie aus dem Konzept der Lewis Acidität, welches dem Fachmann bekannt ist.

Im Rahmen der Erfindung gelten als Grenzfall eingestufte Kationen ("borderline") als harte Kationen.

Die Trifluormethansulfonate sind als harte Anionen klassifiziert in JACS 1972, 94, 856. Dies gilt erfindungsgemäß auch für andere Alkansulfonate und fluorierte Alkansulfonate.

Bevorzugt weisen die als Katalysator eingesetzten Verbindungen wenigstens ein Kation auf, das ausgewählt ist unter Al³⁺, Ti⁴⁺, Cu²⁺, Li⁺, Na⁺, K⁺, Be²⁺, Mg²⁺, Ca²⁺, Sr²⁺, Mn²⁺, Sc³⁺, Ga³⁺, In³⁺, La³⁺, Cr³⁺, Cr⁶⁺, Co³⁺, Fe³⁺, As³⁺, Ir³⁺, Si⁴⁺, Zr⁴⁺, Ce³⁺, Hf⁴⁺, Ge⁴⁺ VO²⁺, (CH₃)Sn²⁺, CH₃Sn³⁺, Fe²⁺, Co²⁺, Ni²⁺, Zn²⁺, Pb²⁺, Sn²⁺, Sb³⁺, Ru²⁺, Os²⁺, Rh³⁺, Bi³⁺, Cl³⁺, Sn⁴⁺,Lu³⁺, MoO³⁺, NO⁺ und WO⁴⁺.

Besonders bevorzugt weisen die als Katalysator eingesetzten Verbindungen wenigstens ein Kation auf, das ausgewählt ist unter Al³⁺, Ti⁴⁺, Cu²⁺, Li⁺, Na⁺, K⁺, Mg²⁺, Ca²⁺, Mn²⁺, Sc³⁺, Co³⁺, Fe³⁺, Si⁴⁺, Zr⁴⁺, Fe²⁺, Co²⁺, Ni²⁺, Zn²⁺, Sn²⁺, Bi³⁺, Sn⁴⁺.

Ganz besonders bevorzugt weisen die als Katalysator eingesetzten Verbindungen wenigstens ein Kation auf, das ausgewählt ist unter Al³⁺, Ti⁴⁺, Cu²⁺.

Bevorzugt weisen die als Katalysator eingesetzten Verbindungen wenigstens ein Anion auf, das ausgewählt ist unter Alkansulfonaten, teilweise fluorierten Alkansulfonaten, vollständig fluorierten Alkansulfonaten, Arensulfonaten, Arylsulfonaten, Acetat, Perchlorat, Nitrat, Fluorid, Sulfat, Carbonat, Azid, Bromid, Phosphat, Nitrit, Sulfit, Hydroxy, O²⁻ und Alkoholaten.

Besonders bevorzugt weisen die als Katalysator eingesetzten Verbindungen wenigstens ein Anion auf, das ausgewählt ist unter Methansulfonat, Ethansulfonat, Propansulfonat, Butansulfonat, Pentansulfonat, Hexansulfonat, Trifluormethansulfonat, Pentafluorethansulfonat, Heptafluorpropansulfonat, Benzolsulfonat, p-Toluolsulfonat, Methanolat, Ethanolat, n-Propanolat, Isopropanolat, n-Butanolat und tert.-Butanolat.

Vorzugsweise ist der Katalysator ausgewählt unter Alkansulfonsäuren und deren Salzen, fluorierten Alkansulfonsäuren und deren Salzen, Arylsulfonsäuren und deren Salzen, Alkyl-Salzen, Alkoholat-Salzen und Kombinationen davon.
Insbesondere vorzugsweise sind die Kationen dieser Salze ausgewählt unter Al³⁺, Ti⁴⁺, Cu²⁺, Li⁺, Na⁺, K⁺, Be²⁺, Mg²⁺, Ca²⁺, Sr²⁺, Mn²⁺, Sc³⁺, Ga³⁺, In³⁺, La³⁺, Cr³⁺, Cr⁶⁺, Co³⁺, Fe³⁺, As³+, Ir³+, Si⁴⁺, Zr⁴⁺, Ce³⁺, Hf⁴⁺, Ge⁴⁺ VO²⁺, (CH₃)Sn²⁺, CH₃Sn³⁺, Fe²⁺, Co²⁺, Ni²⁺, Zn²⁺, Pb²⁺, Sn²⁺, Sb³⁺, Ru²⁺, Os²⁺, Rh³⁺, Bi³⁺, Cl³⁺, Sn⁴⁺,Lu³⁺, MoO³⁺, NO⁺ und WO⁴⁺.

Besonders bevorzugt ist der Katalysator ausgewählt unter Aluminium(III)methansulfonat, Aluminium(III)nonafluorbutansulfonat, Aluminium(III)para-toluolsulfonat, Aluminium(III)trifluormethansulfonat, Kupfer(I)methansulfonat, Kupfer(II)methansulfonat, Kupfer(I)trifluormethansulfonat, Kupfer(II)trifluormethansulfonat, Silbermethansulfonat, Silbertrifluormethansulfonat und Tetra(isopropyl)titanat.

Ganz besonders bevorzugt ist der Katalysator ausgewählt unter Aluminium(III)methansulfonat, Aluminium(III)trifluormethansulfonat, Kupfer(II)methansulfonat, Kupfer(II)trifluormethansulfonat und Tetra(isopropyl)titanat.

In einer weiteren Ausführungsform des Verfahrens zur Herstellung von 2,3,4,5-Tetrahydroxyhexandisäureestern wird der wenigstens eine Katalysator in Schritt b) in einer Menge von 0,0001 Gew.-% bis 10,0 Gew.-%, bevorzugt 0,001 Gew.-% bis 5,0 Gew.-%, besonders bevorzugt 0,01 Gew.-% bis 3,5 Gew.-% und ganz besonders bevorzugt 0,1 bis 2,0 Gew.-%, bezogen auf den Gesamtansatz eingesetzt.

In einer Ausführungsform des Verfahrens, in dem ein Alkohol der Formel (3) als Lösungsmittel eingesetzt wird, kann der wenigstens eine Katalysator in Schritt b) auch in einer Menge von weniger als 0,0001 Gew.-% eingesetzt werden.

In einer bevorzugten Ausführungsform wird das Verfahren in Schritt b) ohne Zusatz eines externen Lösungsmittels durchgeführt. In diesem Falle kann eines der Edukte, beispielsweise die Verbindung der allgemeinen Formel (1) oder die Verbindung der allgemeinen Formel (3), vorzugsweise die Alkoholkomponente der allgemeinen Formel (3), als Lösungsmittel eingesetzt werden.

In einer alternativen Ausführungsform wird das Verfahren in Schritt b) in Gegenwart wenigstens eines von den Verbindungen (2) und (3) verschiedenen Lösungsmittels durchgeführt. Bevorzugt wird die Reaktion dann in Gegenwart eines inerten organischen Lösungsmittels durchgeführt, das vorzugsweise ausgewählt ist unter aliphatischen Ethern, cyclischen Ethern, Ketonen, chlorierten Kohlenwasserstoffen, aliphatischen Kohlenwasserstoffen, cycloaliphatischen Kohlenwasserstoffen, Aromaten und Gemischen davon.

In einer bevorzugten Ausführungsform wird das Verfahren zur Herstellung von 2,3,4,5-Tetrahydroxyhexandisäureestern in Schritt b) ohne Zusatz eines externen Lösungsmittels durchgeführt. Geeignete Lösungsmittel sind THF, Dioxan, Methyl-tert-butylether, Diethylether, Aceton, Methylethylketon, Dichlormethan, Trichlormethan, Tetrachlormethan, Pentan, Hexan, Heptan, Ligroin, Petrolether, Cyclohexan, Benzol, Toluol, Xylol, Chlorbenzol, Dichlorbenzole und Mischungen davon. Bevorzugt wird die Reaktion in Gegenwart eines im Wesentlichen wasserfreien organischen Lösungsmittels durchgeführt.

Die Umsetzung in Schritt b) kann in Abwesenheit oder in Gegenwart eines unter den Reaktionsbedingungen inerten Gases, wie Stickstoff oder Argon, erfolgen.

Das Verhältnis der entstehenden Diester der allgemeinen Formel (1) zu den Monoestern der Formel (1') kann z. B. über die Stöchiometrie der Reaktanden gesteuert werden.

Vorteilhafterweise wird bei der ringöffnenden Umesterung nach dem erfindungsgemäßen Verfahren kein Reaktionswasser gebildet, welches zu einer Erschwerung der Verschiebung des Reaktionsgleichgewichts führen könnte. Auf die in konventionellen Veresterungen übliche Entfernung des bei der Reaktion gebildeten Wassers kann daher verzichtet werden.

Eine Möglichkeit der Quantifizierung des Reaktionsumsatzes kann mittels Gaschromatographie erfolgen. Der Fachmann ist mit dieser Messung vertraut.

Die nach dem erfindungsgemäßen Verfahren erhaltenen rohen Reaktionsgemische können wenigstens einem Aufarbeitungsschritt (Schritt c)) unterzogen werden. Dazu zählen z. B. Neutralisation, Aufreinigung und Trocknung. Eine Aufreinigung kann nach üblichen, dem Fachmann bekannten Methoden erfolgen, z. B. durch Extraktion und/oder Destillation.

In der Regel werden nach dem zuvor beschriebenen Verfahren Reaktionsprodukte erhalten, die Gemische aus Monoestern (1') und Diestern (1) enthalten. Die Herstellung angereicherter oder reiner Diester (1) oder angereicherter oder reiner Monoester (1') gelingt aus den Reaktionsprodukten durch übliche, dem Fachmann bekannte Verfahren (Schritt c)). Dazu zählt beispielsweise die destillative Auftrennung der Reaktionsprodukte.

Ein typisches in Schritt b) erhaltenes Reaktionsprodukt enthält vorzugsweise 100 Gew.-% bis 0 Gew.-%, bevorzugt von 99,99 Gew.-% bis 25 Gew.-%, besonders bevorzugt von 99.9 Gew.-% bis 33 Gew.-% und insbesondere bevorzugt von 99 Gew.-% bis 45 Gew.-% Verbindungen (1), bezogen auf das Gesamtgewicht der Verbindungen (1) und (1').

Ein typisches in Schritt b) erhaltenes Reaktionsprodukt enthält vorzugsweise 0 Gew.-% bis 90 Gew.-%, bevorzugt von 0,01 Gew.-% bis 75 Gew.-%, besonders bevorzugt von 0,1 Gew.-% bis 67 Gew.-% und insbesondere bevorzugt von 1 Gew.-% bis 55 Gew.-% Verbindungen (1'), bezogen auf das Gesamtgewicht der Verbindungen (1) und (1').

Ein weiterer Gegenstand der Erfindung sind Verbindungen der allgemeinen der Formel (1.a),
worin einer der Reste R¹ für C₁-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₈-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht.

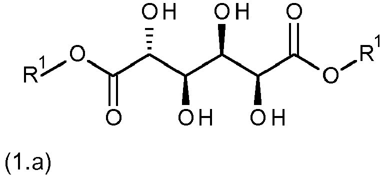

Bevorzugte sind die Reste R¹ in der Verbindung der Formel (1.a) ausgewählt aus den oben genannten bevorzugten Resten R¹.

Weiterhin bevorzugt stehen in der Verbindung (1.a) die Reste R¹ nicht beide gleichzeitig für C₁₈-Alkyl.

Ein weiterer Gegenstand der Erfindung sind Verbindungen der allgemeinen der Formel (1.b),
worin einer der Reste R¹ für C₁-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₃-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht.

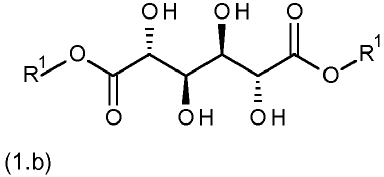

Bevorzugte sind die Reste R¹ in der Verbindung der Formel (1.b) ausgewählt aus den oben genannten bevorzugten Resten R¹.

Weiterhin bevorzugt stehen in der Verbindung (1.b) die Reste R¹ nicht beide gleichzeitig für C₁₈-Alkyl.

Ein weiterer Gegenstand der Erfindung sind Verbindungen der allgemeinen der Formel (1.c),
worin einer der Reste R¹ für C₁-C₂₄-Alkyl, C₂-C₂₄-Alkenyl mit 1, 2, 3 oder mehr als 3 C-C-Doppelbindungen oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₁-C₂₄-Alkyl, C₂-C₂₄-Alkenyl mit 1, 2, 3 oder mehr als 3 C-C-Doppelbindungen oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht.

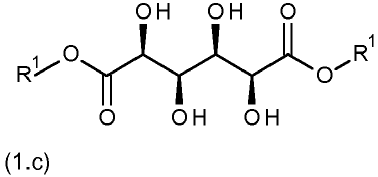

Bevorzugte sind die Reste R¹ in der Verbindung der Formel (1.c) ausgewählt aus den oben genannten bevorzugten Resten R¹.

Weiterhin bevorzugt stehen in der Verbindung (1.c) die Reste R¹ nicht beide gleichzeitig für C₁₈-Alkyl.

Die Verbindungen der allgemeinen Formel (1) eignen sich vorteilhaft als grenzflächenaktive Verbindungen. Sie eignen sich vorteilhaft als Tenside, Emulgatoren und Schaumbildner.

Weiterhin eignen sich Gemische aus wenigstens einer Verbindung der allgemeinen Formeln (1) und gegebenenfalls wenigstens eine Verbindung der allgemeinen Formel (1') vorteilhaft als grenzflächenaktive Verbindungen. Sie eignen sich vorteilhaft als Tenside, Emulgatoren und Schaumbildner.

Die Verbindungen der allgemeinen Formel (1) und Gemische aus Verbindungen der Formeln (1) und (1) eignen sich besonders vorteilhaft als Verdicker und Rheologiemodifizierer, wenn die Reste R¹ unabhängig voneinander ausgewählt sind unter C₁₆-C₂₄-Alkyl, C₁₆-C₂₄-Alkenyl mit 1, 2, 3 oder mehr als 3 C-C-Doppelbindungen und einer Gruppe der Formel -(R²-O)ₙ-R³, worin jedes R² ausgewählt ist unter C₂-C₁₀-Alkylen, R³ für C₁₆-C₂₄-Alkyl oder C₁₆-C₂₄-Alkenyl mit 1, 2, 3 oder mehr als 3 C-C-Doppelbindungen steht und n für eine natürliche Zahl von 1 bis 1000 steht.

Bevorzugt steht R² für C₂-C₄-Alkylen und n für eine natürliche Zahl von 1 bis 500, besonders bevorzugt für eine natürliche Zahl von 2 bis 100 und ganz besonders bevorzugt für eine natürliche Zahl von 4 bis 20.

Die Verbindungen der allgemeinen Formel (1) und Gemische aus Verbindungen der Formeln (1) und (1) eignen sich besonders vorteilhaft als Tenside, wenn die Reste R¹ unabhängig voneinander ausgewählt sind unter C₁₂-C₁₄-Alkyl, C₁₂-C₁₄-Alkenyl mit 1, 2, 3 oder mehr als 3 C-C-Doppelbindungen und einer Gruppe der Formel -(R²-O)ₙ-R³, worin jedes R² ausgewählt ist unter C₂-C₁₀-Alkylen, R³ für C₁₂-C₁₄-Alkyl oder C₁₂-C₁₄-Alkenyl mit 1, 2, 3 oder mehr als 3 C-C-Doppelbindungen steht und n für eine natürliche Zahl von 1 bis 1000 steht.

Bevorzugt steht R² für C₂-C₄-Alkylen und n für eine natürliche Zahl von 1 bis 500, besonders bevorzugt für eine natürliche Zahl von 2 bis 100 und ganz besonders bevorzugt für eine natürliche Zahl von 4 bis 20.

Die Verbindungen der allgemeinen Formel (1) und Gemische aus Verbindungen der Formeln (1) und (1) eignen sich besonders vorteilhaft als Klarspüladditive, wenn die Reste R¹ unabhängig voneinander ausgewählt sind unter C₈-C₁₀-Alkyl, C₈-C₁₀-Alkenyl mit 1, 2, 3 oder mehr als 3 C-C-Doppelbindungen und einer Gruppe der Formel -(R²-O)ₙ-R³ stehen, worin jedes R² ausgewählt ist unter C₂-C₁₀-Alkylen, R³ für C₈-C₁₀-Alkyl oder C₈-C₁₀-Alkenyl mit 1, 2, 3 oder mehr als 3 C-C-Doppelbindungen steht und n für eine natürliche Zahl von 1 bis 1000 steht.

Bevorzugt steht R² für C₂-C₄-Alkylen und n für eine natürliche Zahl von 1 bis 500, besonders bevorzugt für eine natürliche Zahl von 2 bis 100 und ganz besonders bevorzugt für eine natürliche Zahl von 4 bis 20.

Die zuvor genannten als Tenside geeigneten Verbindungen (1) und deren Gemische mit (1') eignen sich auch bevorzugt als Schaumbildner. Sie zeichnen sich durch ein gutes Schaumvermögen aus, d. h. mit ihnen lässt sich zum einen eine gute Höhe des Basisschaums erzielen, andererseits verfügen sie auch über eine gute Schaumbeständigkeit, insbesondere in hartem Wasser. Bezüglich geeigneter und bevorzugter Schaumbildner wird auf die geeigneten und bevorzugten Tenside in vollem Umfang Bezug genommen.

Die Verbindungen der allgemeinen Formel (1) und Gemische, die zusätzlich wenigstens eine Verbindung der allgemeinen Formel (1') enthalten, eignen sich in vorteilhafter Weise zur Modifizierung der rheologischen Eigenschaften wässriger Zusammensetzungen. Es kann sich ganz allgemein beispielsweise um kosmetische Zusammensetzungen, pharmazeutische Zusammensetzungen, Hygieneprodukte, Anstrichmittel, Zusammensetzungen für die Papierindustrie sowie die Textilindustrie handeln.

Die Verbindungen der allgemeinen Formel (1) und Gemische, die zusätzlich wenigstens eine Verbindung der allgemeinen Formel (1') enthalten, eignen sich bevorzugt zur Verdickung der Konsistenz von tensidhaltigen wässrigen Zusammensetzungen in einem weiten Bereich. Sie fungieren dabei speziell als sogenannte "mizellare Verdicker", d. h. grenzflächenaktive Verbindungen, die zur Erhöhung der Viskosität von tensidhaltigen Formulierungen eingesetzt werden. Je nach Grundkonsistenz der flüssigen Zusammensetzungen können in Abhängigkeit von der Einsatzmenge des Copolymers in der Regel Fließeigenschaften von dünnflüssig bis hin zu fest (im Sinne von "nicht mehr fließend") erzielt werden.

Als Rheologiemodifizierer eignen sich bevorzugt Verbindungen der allgemeinen Formel (1) und Gemische, die zusätzlich wenigstens eine Verbindung der allgemeinen Formel (1') enthalten, worin die Reste R¹ unabhängig voneinander ausgewählt sind unter C₁₆-C₂₄-Alkyl, C₁₆-C₂₄-Alkenyl mit 1, 2, 3 oder mehr als 3 C-C-Doppelbindungen und einer Gruppe der Formel -(R²-O)ₙ-R³, worin jedes R² ausgewählt ist unter C₂-C₁₀-Alkylen, R³ für C₁₆-C₂₄-Alkyl oder C₁₆-C₂₄-Alkenyl mit 1, 2, 3 oder mehr als 3 C-C-Doppelbindungen steht und n für eine natürliche Zahl von 1 bis 1000 steht. Bevorzugt steht R² für C₂-C₄-Alkylen und n für eine natürliche Zahl von 1 bis 500, besonders bevorzugt für eine natürliche Zahl von 2 bis 100 und ganz besonders bevorzugt für eine natürliche Zahl von 4 bis 20.

Die Verbindungen der allgemeinen Formel (1) und Gemische, die zusätzlich wenigstens eine Verbindung der allgemeinen Formel (1') enthalten, eignen sich besonders vorteilhaft zur Bereitstellung von Formulierungen, speziell kosmetischen oder pharmazeutischen Zusammensetzungen sowie Wasch-, Reinigungs- oder Geschirrspülmitteln, die wenigstens ein von den Verbindungen der allgemeinen Formeln (1) und (1') verschiedenes Tensid enthalten. Insbesondere handelt es sich dabei um wässrige Formulierungen. Die Verbindungen (1) und deren Gemische mit (1') zeichnen sich in derartigen Formulierungen durch wenigstens eine der folgenden Eigenschaften aus: eine gute oberflächenaktive Wirkung, eine gute Verdickungswirkung auch bei geringen Einsatzmengen, und eine gute Verträglichkeit mit weiteren Tensiden.

Geeignete zusätzliche Tenside sind anionische Tenside, nichtionische Tenside, kationische Tenside, amphotere Tenside und Mischungen davon.

Typische Beispiele für anionische Tenside sind Seifen, Alkylsulfonate, Alkylbenzolsulfonate, Olefinsulfonate, Alkylethersulfonate, Glycerinethersulfonate, Methylestersulfonate, Sulfofettsäuren, Alkylsulfate, Ethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acylglutamate und Acylaspartate, sowie Acyllactylate, Acyltartrate, Alkyloligoglucosidsulfate, Alkylglucosecarboxylate, Proteinfettsäurekondensate und Alkyl(ether)phosphate.

Geeignete Seifen sind z. B. Alkali-, Erdalkali- und Ammoniumsalze von Fettsäuren, wie Kaliumstearat.

Bevorzugte Alkylsulfate sind Sulfate von Fettalkoholen der allgemeinen Formel R³-O-SO₃Y¹, worin R³ für einen linearen oder verzweigten, gesättigten oder ungesättigten Kohlenwasserstoffrest mit 6 bis 22 Kohlenstoffatomen und Y¹ für ein Alkalimetall, das einwertige Ladungsäquivalent eines Erdalkalimetalls, Ammonium-, Mono-, Di-, Tri- oder Tetraalkylammonium, Alkanolammonium oder Glucammonium steht. Geeignete Fettalkoholsulfate werden vorzugsweise durch Sulfatierung von nativen Fettalkoholen oder synthetischen Oxoalkoholen und nachfolgender Neutralisation erhalten werden. Typische Beispiele für Fettalkoholsulfate sind die Sulfatierungsprodukte des Capronalkohols, Caprylalkohols, 2-Ethylhexylalkohols, Caprinalkohols, Laurylalkohols, Isotridecylalkohols, Myristylalkohols, Cetylalkohols, Palmoleylalkohols, Stearylalkohols, Isostearylalkohols, Oleylalkohols, Elaidylalkohols, Petroselinylalkohols, Linolylalkohols, Linolenylalkohols, Behenylalkohols und Elaeostearylalkohols sowie die Salze und Mischungen davon. Bevorzugte Salze der Fettalkoholsulfate sind die Natrium- und Kaliumsalze, insbesondere die Natriumsalze. Bevorzugte Mischungen der Fettalkoholsulfate basieren auf technischen Alkoholmischungen, die z. B. bei der Hochdruckhydrierung von technischen Methylestern auf Basis von Fetten und Ölen oder bei der Hydrierung von Aldehyden aus der Oxosynthese oder bei der Dimerisierung von ungesättigten Fettalkoholen anfallen. Vorzugsweise werden zur Herstellung von Alkylsulfaten Fettalkohole und Fettalkoholgemische mit 12 bis 18 Kohlenstoffatomen und insbesondere 16 bis 18 Kohlenstoffatomen eingesetzt. Typische Beispiele hierfür sind technische Alkoholsulfate auf Basis von pflanzlichen Rohstoffen.

Eine bevorzugte Klasse anionischer Tenside sind die Ethersulfate. Ethersulfate (Alkylethersulfate) stellen bekannte anionische Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden können.

Bevorzugt sind insbesondere Fettalkoholethersulfate der allgemeinen Formel R⁶O-(CH₂CH₂O)ₘSO₃Y², in der R⁶ für einen linearen oder verzweigten Alkyl- und/oder Alkenylrest mit 6 bis 22 Kohlenstoffatomen, m für Zahlen von 1 bis 10 und Y² für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze.

Zu den nichtionischen Tensiden gehören beispielsweise:
- Fettalkoholpolyoxyalkylenester, beispielsweise Laurylalkoholpolyoxyethylenacetat,
- Alkylpolyoxyalkylenether, die sich von niedermolekularen C₁-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Dazu zählen speziell Fettalkoholalkoxylate und Oxoalkoholalkoxylate, insbesondere vom Typ RO-(R⁸O)ᵣ(R⁹O)ₛR¹⁰ mit R⁸ und R⁹ unabhängig voneinander = C₂H₄, C₃H₆, C₄H₈ und R¹⁰ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie isoTridecylalkohol- und Oleylalkoholpolyoxyethylenether,
- Alkylarylalkohol-polyoxyethylenether, z. B. Octylphenol-polyoxyethylenether,
- Zuckertenside, bevorzugt Alkylpolyglycoside, Sorbitester, wie beispielsweise Sorbitanfettsäureester (Sorbitanmonooleat, Sorbitantristearat), Polyoxyethylensorbitanfettsäureester, N-Alkylgluconamide,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Alkylmethylsulfoxide,
- Alkyldimethylphosphinoxide, wie beispielsweise Tetradecyldimethylphosphinoxid.

Eine bevorzugte Klasse nichtionischer Tenside sind die Zuckertenside, speziell Alkyl-(poly)glycoside. Im Sinne der Erfindung wird der Begriff Alkyl(poly)glycoside synonym zu Alkyl(oligo)glycoside verwendet und auch mit der Abkürzung "APG" bezeichnet. Alkylglycoside und/oder Alkylpolyglycoside umfassen sowohl Alkyl- als auch Alkenyl(poly)-glycoside und haben bevorzugt die Formel R¹¹O-[G]ₚ, in der R¹¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p gibt den Polymerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylpolyglykosid eine analytisch ermittelte rechnerische Größe, die meistens eine gebrochene Zahl darstellt. Vorzugsweise werden Alkyl- und/oder Alkenylpolyglykoside mit einem mittleren Polymerisierungsgrad p von 1,1 bis 3,0 eingesetzt. Aus anwendungstechnischer Sicht sind solche Alkyl- und/oder Alkenylpolyglykoside bevorzugt, deren Polymerisierungsgrad kleiner als 1,7 ist und insbesondere zwischen 1,2 und 1,4 liegt.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder-dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den kationischen Tensiden gehören beispielsweise quaternierte Ammoniumverbindungen, insbesondere Alkyltrimethylammonium- und Dialkyldimethylammonium-halogenide und -alkylsulfate sowie Pyridin- und Imidazolin-Derivate, insbesondere Alkylpyridinium-Halogenide. Beispielsweise können Behenyl oder Cetyltrimethylammoniumchlorid eingesetzt werden. Geeignet sind weiterhin sogenannte Esterquats, die auf quartären Triethanol-Methyl-Ammonium- oder quartären Diethanol-Dimethyl-Ammonium-Verbindungen mit langen Kohlenwasserstoffketten in Form von Fettsäureestern basieren. Dazu zählt beispielsweise Bis(acyloxyethyl)hydroxyethylammonium Methosulfat. Geeignet ist weiterhin Dehyquart L 80 (INCI: Dicocoylethyl Hydroxyethylmonium Methosulfate (and) Propylene Glycol).

Die Verbindungen der allgemeinen Formel (1) und Gemische, die zusätzlich wenigstens eine Verbindung der allgemeinen Formel (1') enthalten, eignen sich vorzugsweise zur Formulierung von kosmetischen und pharmazeutischen Zusammensetzungen, speziell von wässrigen kosmetischen und pharmazeutischen Zusammensetzungen.

Ein weiterer Gegenstand der Erfindung sind kosmetische oder pharmazeutische Zusammensetzungen, enthaltend wenigstens eine Verbindung der allgemeinen Formel (1), gegebenenfalls wenigstens eine Verbindung (1') und wenigstens einen davon verschiedenen kosmetischen oder pharmazeutischen Wirkstoff und/oder Hilfsstoff.

Bevorzugt enthalten die erfindungsgemäßen kosmetischen und pharmazeutischen Zusammensetzungen die Verbindungen der allgemeinen Formel (1) und, falls vorhanden (1') in einer Gesamtmenge von 0,1 bis 50 Gew.-%, besonders bevorzugt von 0,5 bis 30 Gew.-%, bezogen auf das Gesamtgewicht des Mittels.

Bevorzugt enthalten die erfindungsgemäßen kosmetischen und pharmazeutischen Zusammensetzungen als Hilfsstoff wenigstens einen kosmetisch oder pharmazeutisch akzeptablen Träger.

Vorzugsweise ist der kosmetisch oder pharmazeutisch akzeptable Träger ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Speziell geeignete kosmetisch verträgliche Öle, Fette und Wachse sind in Karl-Heinz Schrader, Grundlagen und Rezepturen der Kosmetika, 2. Auflage, Verlag Hüthig, Heidelberg, S. 319 - 355 (1989) beschrieben, worauf hier Bezug genommen wird.

Bevorzugte Öle, Fette und Wachse sind mineralische und synthetische Öle, wie z. B. Paraffine und aliphatische Kohlenwasserstoffe mit mehr als 8 Kohlenstoffatomen, Purcellinöl, Perhydrosqualen, Siliconöle, natürliche (tierische oder pflanzliche) Öle und Fette, wie z. B. Sonnenblumenöl, Kokosöl, Palmkernöl, Palmöl, Sojaöl, Avocadoöl, Olivenöl, Süßmandelöl, Calophylumöl, Ricinusöl, Sesamöl, Jojobaöl, Karite-Öl, Hoplostethus-ÖI, Lanolin und Derivate davon (z. B. hydriertes Lanolin und acetyliertes Lanolin), oder Wachse, Fettsäuren, Fettsäureester, wie z. B. Triglyceride von C₆-C₃₀-Fettsäuren, Wachsester, Fettalkohole, Vaseline, sowie Mischungen davon. Geeignete Wachse sind z. B. Carnaubawachs, Candilillawachs, Bienenwachs, mikrokristallines Wachs, Ozokeritwachs und Ca-, Mg- und Al-Oleate, -Myristate, -Linoleate und -Stearate. Derartige kosmetisch verträgliche Öle, Fette und Wachse werden speziell in hautkosmetischen und dermatologischen Zusammensetzungen eingesetzt.

Bei den erfindungsgemäßen Zusammensetzungen kann es sich um hautkosmetische, haarkosmetische, dermatologische, hygienische oder pharmazeutische Zusammensetzungen handeln. Vorteilhafterweise eignen sich die Verbindungen der allgemeinen Formel (1) und deren Gemische mit (1') aufgrund der zuvor beschriebenen Eigenschaften für den Einsatz in einer Vielzahl verschiedener kosmetischer oder pharmazeutischer Zusammensetzungen. Sie können dabei als Wirkstoff, als Hilfsstoff oder als Komponente mit einer Mehrfachwirkung zum Einsatz kommen. So eignen sich die Verbindungen (1) und deren Gemische mit (1') z. B. im Bereich der Reinigung von Haut und Haaren für Zusammensetzungen, die wenigstens eine der folgenden Eigenschaften aufweisen: eine gute Reinigungsleistung; gute rheologische Eigenschaften, d. h. die Zusammensetzungen sind zwar fließfähig, aber nicht zu leicht- oder zähflüssig, um eine optimale Applikation zu ermöglichen; die Fähigkeit, der Haut Flüssigkeit zuzuführen oder rückfettend zu wirken.

Die Verbindungen (1) und deren Gemische mit (1') eignen sich auch bevorzugt als Schaumbildner in kosmetischen Zusammensetzungen, insbesondere Mitteln zur Pflege und/oder Reinigung der Haare.

In einer speziellen Ausführungsform handelt es sich bei den erfindungsgemäßen Zusammensetzungen um ein Reinigungsmittel für die Haut und/oder die Haare. Diese enthalten vorzugsweise wenigstens eine der zuvor beschriebenen Verbindungen der Formel (1) und gegebenenfalls zusätzliche wenigstens eine Verbindung der Formel (1') mit der Eignung als Tenside.

In einer weiteren speziellen Ausführungsform handelt es sich bei den erfindungsgemäßen Zusammensetzungen um ein Mittel zur Pflege und/oder zum Schutz der Haut. Diese können dann wenigstens eine der zuvor beschriebenen Verbindungen der Formel (1) und deren Gemische mit (1') enthalten. Diese können weiterhin wenigstens einen schwerlöslichen Wirkstoff, z. B. einen UV-Filter, und wenigstens eine der zuvor beschriebenen Verbindungen der Formel (1) enthalten.

In einer weiteren speziellen Ausführungsform handelt es sich bei den erfindungsgemäßen Zusammensetzungen um ein Mittel für die dekorative Kosmetik. Eine weitere spezielle Ausführungsform sind Haut- und Haarconditioner, die wenigstens eine Verbindung der Formel (1) und gegebenenfalls zusätzlich wenigstens eine Verbindung der Formel (1') enthalten.

Aufgrund ihrer verdickenden Eigenschaften eignen sich die zuvor beschriebenen Verbindungen der Formel (1) und deren Gemische mit (1') insbesondere auch als Zusatzstoffe für Haar- und Hautkosmetika.

Vorzugsweise liegen die erfindungsgemäßen Zusammensetzungen in Form einer Creme, Mousse, Milch, Lotion, Mascara, Theaterfarbe, Seife von flüssiger bis fester Konsistenz, eines Schaums, Gels, Sprays, Stifts, Wasch-, Dusch- oder Badepräparats von flüssiger bis gelförmiger Konsistenz, Lidschattens, Eyeliners, Rouges, Puders oder Strips vor. Gewünschtenfalls können auch Liposomen oder Mikrosphären eingesetzt werden.

Die erfindungsgemäßen kosmetischen Zusammensetzungen können zusätzlich kosmetisch und/oder dermatologisch aktive Wirk- und Effektstoffe sowie Hilfsstoffe enthalten. Vorzugsweise enthalten die erfindungsgemäßen kosmetischen Mittel wenigstens eine Verbindung der Formel (1), wie vorstehend definiert, gegebenenfalls zusätzlich wenigstens eine Verbindung der Formel (1'), wie vorstehend definiert, wenigstens einen wie vorstehend definierten Träger c) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Zusätzlich zu den Verbindungen der Formel (1) und gegebenenfalls (1') können die kosmetischen Mittel wenigstens ein konventionelles Verdickungsmittel enthalten. Dazu zählen z. B. Polysaccharide und organische Schichtmineralien wie Xanthan Gum® (Kelzan® der Fa. Kelco), Rhodopol® 23 (Rhone Poulenc) oder Veegum® (Firma R. T. Vanderbilt) oder Attaclay® (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmineralien wie Montmorillonite, Zeolithe, Kieselsäuren).

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Die erfindungsgemäßen kosmetischen Zusammensetzungen können als kosmetischen Wirkstoff (wie auch gegebenenfalls als Hilfsstoff) wenigstens ein kosmetisch oder pharmazeutisch akzeptables Polymer enthalten. Dazu zählen ganz allgemein anionische, kationische, amphotere und neutrale Polymere.

Nach einer bevorzugten Ausführungsform handelt es sich bei den erfindungsgemäßen Zusammensetzungen um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Zusammensetzungen um kosmetische Zusammensetzungen zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Bei den erfindungsgemäßen Hautpflegemitteln handelt es sich insbesondere um W/O- oder O/W-Hautcremes, Tag- und Nachtcremes, Augencremes, Gesichtscremes, Antifa-Itencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Verbindungen der Formel (1) und deren Gemische mit (1') zeigen vorteilhafte Wirkungen. Die Verbindungen der Formel (1) und deren Gemische mit (1') können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Zusammensetzungen enthalten vorzugsweise wenigstens eine Verbindungen der Formel (1) und gegebenenfalls zusätzlich wenigstens eine Verbindungen der Formel (1') in einer Gesamtmenge von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Je nach Anwendungsgebiet können die erfindungsgemäßen Zusammensetzungen in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zusammensetzungen können neben den Verbindungen der Formel (1) und deren Gemischen mit (1') sowie geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Zur Einstellung bestimmter Eigenschaften, wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zusammensetzungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zusammensetzungen erfolgt nach üblichen, dem Fachmann bekannten Verfahren.

Nach einer weiteren bevorzugten Ausführungsform handelt es sich bei den erfindungsgemäßen Mitteln um ein Duschgel, eine Shampoo-Formulierung oder ein Badepräparat. Solche Formulierungen enthalten wenigstens eine Verbindung der allgemeinen Formel (1), gegebenenfalls wenigstens eine Verbindung der allgemeinen Formel (1') sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln. Geeignete Tenside sind die zuvor genannten.

Die Verbindungen der allgemeinen Formel (1) und deren Gemische mit (1') eignen sich vorteilhaft für die Verwendung in Wasch- und Reinigungsmitteln, in Geschirrspülmittel und in Klarspülern.

Sie eignen sich insbesondere in Klarspülformulierungen für die maschinelle Geschirrreinigung (adw = automatic dish washing). Die erfindungsgemäß verwendeten Verbindungen der allgemeinen Formel (1) und deren Gemische mit (1') haben sowohl eine Wirkung als Tensid als auch als Cobuilder. Sie eignen sich zur Herstellung von Maschinengeschirrspülmitteln, Klarspülmitteln und Maschinengeschirrspülmitteln mit Klarspülfunktion. Die Formulierungen für die maschinelle Geschirrreinigung zeichnen sich insbesondere durch eine hervorragende belagsinhibierende Wirkung beim Einsatz im Klarspülgang des maschinellen Geschirrspülers aus (d. h. sie wirkt als Inkrustierungsinhibitor). Sie wirken dabei sowohl gegenüber anorganischen als auch gegenüber organischen Belägen inhibierend. Zudem verhindern sie die Flecken- und Tropfenbildung.

Beispiele für Reinigungsmittel, die die Verbindungen der allgemeinen Formel (1) und deren Gemische mit (1') enthalten, umfassen Wasch- und Reinigungsmittel, Geschirrspülmittel, wie Handgeschirrspülmittel oder Maschinengeschirrspülmittel (= Geschirrspülmittel für die Geschirrspülmaschine), Metallentfetter, Glasreiniger, Fußbodenreiniger, Allzweckreiniger, Hochdruckreiniger, Neutralreiniger, alkalische Reiniger, saure Reiniger, Spritzentfetter, Molkereireiniger, Großküchenreiniger, Apparatereiniger in der Industrie, insbesondere der chemischen Industrie, Reiniger für der Autowäsche und auch Haushalts-Allzweckreiniger.

Ein weiterer Gegenstand der Erfindung sind Wasch-, Reinigungs-, Geschirrspülmittel oder Klarspüler die wenigstens eine Verbindung der allgemeinen Formel (1) und gegebenenfalls wenigstens eine Verbindung der allgemeinen Formel (1') enthalten.

Dabei kann die Verbindung der allgemeinen Formel (1) und deren Gemische mit (1') sowohl als grenzflächenaktive Verbindung als auch als Rheologiemodifizierer eingesetzt werden. Auf die zuvor beschriebenen geeigneten und bevorzugten Verbindungen (1) und (1') für die Verwendung als grenzflächenaktive Verbindung und als Rheologiemodifizierer wird hier in vollem Umfang Bezug genommen.

Das erfindungsgemäße Wasch-, Reinigungs- und Geschirrspülmittel umfasst vorzugsweise folgende Bestandteile:
a) wenigstens eine Verbindung der allgemeinen Formel (1) und gegebenenfalls wenigstens eine Verbindung der allgemeinen Formel (1');
b) wenigstens einen Builder (auch als Sequestrierungsmittel, Gerüststoff, Komplexbildner, Chelator, Chelatisierungsmittel oder Enthärter bezeichnet);
c) gegebenenfalls wenigstens ein Enzym;
d) gegebenenfalls wenigstens ein Bleichmittel; und
e) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Tablettierhilfsmitteln, Desintegrationsmiteln, Verdickern, Löslichkeitsvermittlern, organischen Lösemitteln, Elektrolyten, pH-Stellmitteln, Parfümträgern, Fluoreszenzmitteln, Hydrotropen, Antiredepositionsmitteln, optischen Aufhellern, Vergrauungsinhibitoren, Einlaufverhinderern, Knitterschutzmitteln, Farbübertragungsinhibitoren, antimikrobiellen Wirkstoffen, Antioxidantien, Korrosionsinhibitoren, Antistatika, Bügelhilfsmitteln, Phobier- und Imprägniermitteln, Quell- und Schiebefestmitteln, UV-Absorbern und Wasser.

Vorzugsweise enthalten die erfindungsgemäßen Wasch- und Reinigungsmittel:
a) wenigstens eine Verbindung der allgemeinen Formel (1) und gegebenenfalls wenigstens eine Verbindung der allgemeinen Formel (1'): von 0,1 bis 20 Gew.-%;
b) wenigstens ein Builder: von 5 bis 80 Gew.-%;
c) wenigstens ein Enzym: von 0 bis 8 Gew.-%;
d) wenigstens ein Bleichmittel: von 0 bis 30 Gew.-%; und
e) wenigstens ein weiterer Zusatzstoff: 0 bis 50 Gew.-%.

Die Gew.-%-Angaben beziehen sich dabei auf das Gesamtgewicht des Wasch- und Reinigungsmittels. Die Gewichtsmengen von a) bis e) ergänzen sich zu 100 Gew.-%.

Eine erfindungsgemäße Geschirrreinigungszusammensetzung umfasst vorzugsweise folgende Bestandteile:
a) wenigstens eine Verbindung der allgemeinen Formel (1) und gegebenenfalls wenigstens eine Verbindung der allgemeinen Formel (1'),
b) wenigstens einen Builder (auch als Sequestrierungsmittel, Gerüststoff, Komplexbildner, Chelator, Chelatisierungsmittel oder Enthärter bezeichnet),
c) gegebenenfalls wenigstens ein Enzym,
d) gegebenenfalls wenigstens ein Bleichmittel,
e1) Wasser,
e2) gegebenenfalls wenigstens einen Verdicker, und
e3) gegebenenfalls wenigstens einen weiteren Zusatzstoff, der vorzugsweise ausgewählt ist unter von a) verschiedenen Tensiden, Basen, Korrosionsinhibitoren, Entschäumern, Farbstoffen, Duftstoffen, Füllstoffen, Löslichkeitsvermittlern und organischen Lösungsmitteln.

Die erfindungsgemäße Zusammensetzung für die Geschirrreinigung enthält, bezogen auf das Gesamtgewicht der Zusammensetzung, vorzugsweise:
a) 0,1 bis 50 Gew.-% wenigstens einer erfindungsgemäßen Verbindung der Formel (1) und gegebenenfalls wenigstens eine Verbindung der allgemeinen Formel (1'),
b) 5 bis 90 Gew.-% wenigstens eines Builders und/oder Cobuilders,
c) 0 bis 8 Gew.-% wenigstens eines Enzyms,
d) 0 bis 30 Gew.-% wenigstens eines Bleichmittels,
e1) 0,1 bis 90 Gew.-% Wasser,
e2) 0 bis 8 Gew.-% wenigstens eines Verdickers
e3) 0 bis 25 Gew.-% wenigstens eines weiteren Zusatzstoffs,
mit der Maßgabe, dass sich die Gewichtsmengen der Komponenten a) bis e) zu 100 Gew.-% ergänzen.

Allgemein können die erfindungsgemäßen Verbindungen der allgemeinen Formel (1) und deren Gemische mit (1') in allen Bereichen eingesetzt werden, in denen eine verdickende Wirkung in Kombination mit grenzflächenaktiven Stoffen notwendig ist.

Weiterhin sind die Verbindungen der allgemeinen Formel (1) und deren Gemische mit (1') geeignet, die Löslichkeit anderer Komponenten, z. B. anderer oberflächenaktiver Komponenten, wie von anionischen Tensiden, zu verbessern. Sie tragen somit auch positiv zur Bildung klarer tensidhaltiger Lösungen bei.

Wirkstoffe für die Kosmetik, Arzneimittel, den Pflanzenschutz und für den Materialschutz, d. h. Substanzen, die auch in geringer Konzentration bereits eine Wirkung entfalten, z. B. eine kosmetische Wirkung, eine pharmakologische Wirkung in einem Organismus, eine physiologische Wirkung in einer Pflanze oder einem Schadorganismus, etc., werden häufig in Form flüssiger, speziell wässriger, Zusammensetzungen formuliert und angewendet. Alternativ ist auch eine Formulierung und Verabreichung in fester Form, z. B. als Pulver oder Pressling (Tablette, etc.) möglich, wobei der Transport an den eigentlichen Wirkort jedoch die Umwandlung in eine flüssige, speziell wässrige, Form umfasst.

Die Erfindung wird anhand der folgenden nicht einschränkenden Beispiele näher erläutert.

### BEISPIELE

Die folgenden Beispiele dienen der Verdeutlichung der Erfindung, ohne sie in irgendeiner Weise zu beschränken.

Es wurden folgende Abkürzungen verwendet:
Äq. für Äquivalent,
MeOH für Methanol,
MIBK für Methylisobutylketon,
OAc für Acetat,
OiPr für iso-Propylat,
OMs für Methansulfonat,
OTf für Trifluormethansulfonat,
Temp. für Temperatur,
THF für Tetrahydrofuran.

HD-Ocenol 90/95 V ist ein Gemisch beinhaltend Oleylalkohol mit einer C-Ketten-Verteilung von 0-2,0 % C14, 2,0-10,0 % C16, 90,0-98,0 % C18, 0-3,0 % >C18; HD-Ocenol ist eine eingetragene Marke der Firma BASF.

Lorol Spezial ist ein Gemisch beinhaltend Laurylalkohol und Myristylalkohol mit einer C-Ketten-Verteilung von 0-2,0 % C10, 70,0-75,0 % C12, 24,0-30,0 % C14, 0-2,0 % C16; Lorol ist eine eingetragene Marke der Firma BASF.

### Beispiel 1

In einem 50 mL Glasgefäß mit Magnetrührstäbchen wurden Glucarodilacton (1,0 Äq., 3,50 g, 20 mmol), 1-Decanol (2,0 Äq., 9,50 g, 60 mmol) und Aluminium(III)methansulfonat (0,02 Äq., 0,13 g, 0,4 mmol) vorgelegt. Die Reaktionsmischung wurde unter Rühren langsam auf 60 °C erwärmt, wobei das Dilacton in Lösung ging. Nach 5 h bei 60 °C wurde die Zusammensetzung der Produktmischung gaschromatographisch bestimmt. Analyse des Rohprodukts: 24,9 % 1-Decanol, 19,3 % Glucarsäurelacton-Monodecylester und 50,4 % Glucarsäure-Didecylester.

### Weitere Beispiele

Die in Tabelle 1, Eintrag 1 bis 21 beschriebenen Beispiele wurden analog der Versuchsbeschreibung aus Beispiel 1 durchgeführt.

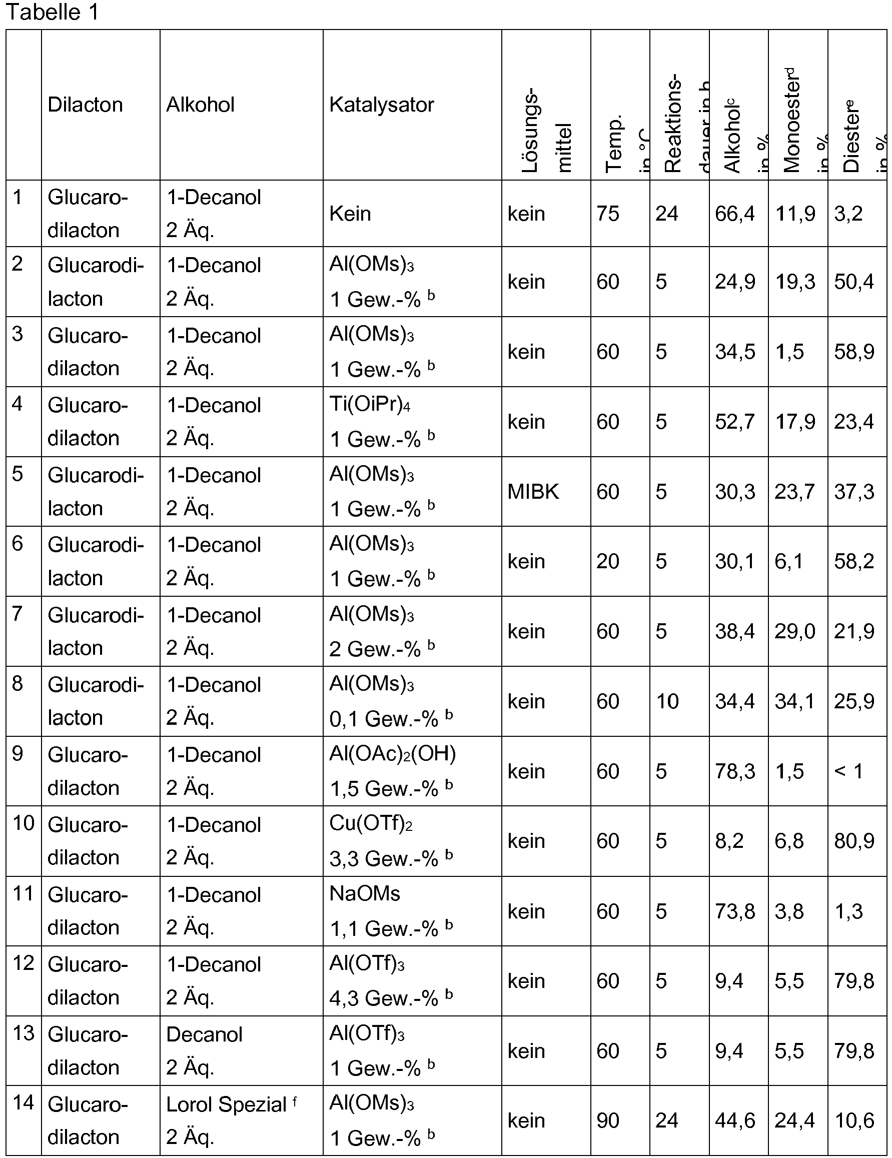

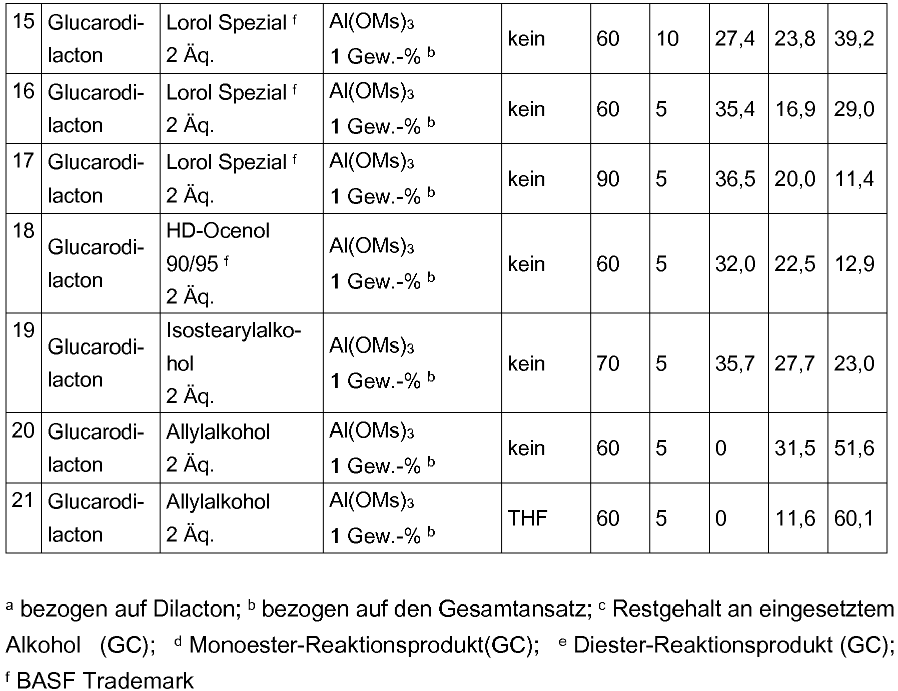

## Patentansprüche

1. Verfahren zur Herstellung von 2,3,4,5-Tetrahydroxyhexandisäureestern umfassend wenigstens eine Verbindung der allgemeinen Formel (1), 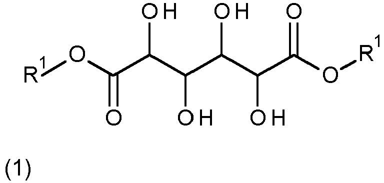 worin die Reste R¹ unabhängig voneinander für C₁-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ stehen, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
bei dem man
a) eine Verbindung der Formel (2) 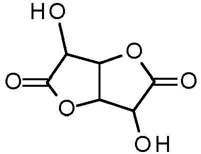 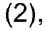 die ausgewählt ist unter Verbindungen der Formeln (2.a), (2.b) und (2.c). 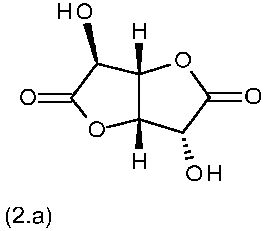 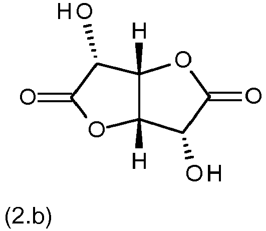 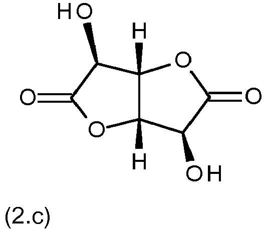 bereitstellt, und
b) die in Schritt a) bereitgestellte Verbindung der Formel (2) mit wenigstens einer Verbindung der allgemeinen Formel (3),
R¹-OH (3)
worin R¹ die oben angegebene Bedeutung besitzt,
in Gegenwart wenigstens eines Katalysators umsetzt,
welcher ausgewählt ist unter Verbindungen, die wenigstens ein Lewis-saures, hartes Kation und/oder wenigstens ein hartes Anion aufweisen.

2. Verfahren nach Anspruch 1, wobei die 2,3,4,5-Tetrahydroxyhexandisäureester zusätzlich wenigstens eine Verbindung der allgemeinen Formel (1') umfassen 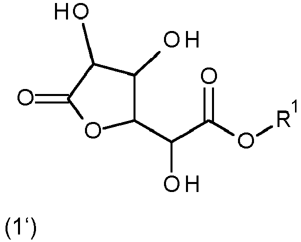 worin R¹ wie in Anspruch 1 definiert ist.

3. Verfahren nach einem der vorherigen Ansprüche, wobei in Schritt b) die Gesamtmenge an Verbindungen (3) bezogen auf die Gesamtmenge der Verbindung (2) 0,5 bis 100 Moläquivalente, bevorzugt 0,5 bis 50 Moläquivalente, besonders bevorzugt 1 bis 10 Moläquivalente und ganz besonders bevorzugt 1 bis 4 Moläquivalente beträgt.

4. Verfahren nach einem der vorherigen Ansprüche, wobei die Umsetzung in Schritt b) bei einer Temperatur in einem Bereich von 10 bis 150 °C, bevorzugt in einem Bereich von 15 bis 90 °C und besonders bevorzugt in einem Bereich von 20 bis 70 °C, durchgeführt wird.

5. Verfahren nach einem der vorherigen Ansprüche, wobei in Schritt b) der Katalysator ausgewählt ist unter Verbindungen, die wenigstens ein Kation enthalten, welches ausgewählt ist unter, Al3+, Ti4+, Cu2+, Li+, Na+, K+, Be2+, Mg2+, Ca2+, Sr2+, Mn²⁺, Sc³⁺, Ga³⁺, In³⁺, La³⁺, Cr³⁺, Cr⁶⁺, Co³⁺, Fe³⁺, As³+, Ir³+, Si⁴⁺, Zr⁴⁺, Ce³⁺, Hf⁴⁺, Ge⁴⁺ VO²⁺, (CH₃)Sn²⁺, CH₃Sn³⁺, Fe²⁺, Co²⁺, Ni²⁺, Zn²⁺, Pb²⁺, Sn²⁺, Sb³⁺, Ru²⁺, Os²⁺, Rh³⁺, Bi³⁺, Cl³+, Sn⁴⁺,Lu³⁺, MoO³⁺, NO⁺ und WO⁴⁺.

6. Verfahren nach einem der vorherigen Ansprüche, wobei in Schritt b) der Katalysator ausgewählt ist unter Verbindungen, die wenigstens ein Anion enthalten, welches ausgewählt unter Alkansulfonaten, teilweise fluorierten Alkansulfonaten, vollständig fluorierten Alkansulfonaten, Arensulfonaten, Arylsulfonaten, Acetat, Perchlorat, Nitrat, Fluorid, Sulfat, Carbonat, Azid, Bromid, Phosphat, Nitrit, Sulfit, Hydroxy, O²⁻ und Alkoholaten.

7. Verfahren nach einem der vorherigen Ansprüche, wobei in Schritt b) der Katalysator ausgewählt ist unter Aluminium(III)methansulfonat, Aluminium(III)nonafluorbutansulfonat, Aluminium(III)para-toluolsulfonat, Aluminium(III)trifluormethansulfonat, Kupfer(I)methansulfonat, Kupfer(II)methansulfonat, Kupfer(I)trifluormethansulfonat, Kupfer(II)trifluormethansulfonat, Silbermethansulfonat, Silbertrifluormethansulfonat und Tetra(isopropyl)titanat.

8. Verfahren nach einem der vorherigen Ansprüche, wobei die Umsetzung in Schritt b) ohne Zusatz eines externen Lösungsmittels oder in Gegenwart wenigstens eines von den Verbindungen R¹⁻OH verschiedenen Lösungsmittel durchgeführt wird, das ausgewählt ist unter aliphatischen Ethern, cyclischen Ethern, Ketonen, chlorierten Kohlenwasserstoffen, aliphatischen Kohlenwasserstoffen, cycloaliphatischen Kohlenwasserstoffen, Aromaten und Gemischen davon.

9. Verbindungen der allgemeinen Formel (1), ausgewählt unter
Verbindungen der Formel (1.a), 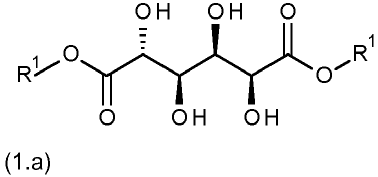
worin einer der Reste R¹ für C₁-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₂-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₈-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₁-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht;
Verbindungen der Formel (1.b), 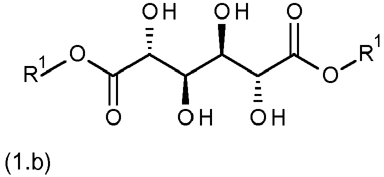
worin einer der Reste R¹ für C₁-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₁-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₃-C₂₄-Alkyl, C₂-C₂₄-Alkenyl mit 1, 2, 3 oder mehr als 3 C-C-Doppelbindungen, oder für eine Gruppe der Formel -(R²-O)ₙ-R³ steht, worin jedes R² ausgewählt ist unter C₁-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht; und
Verbindungen der Formel (1.c), 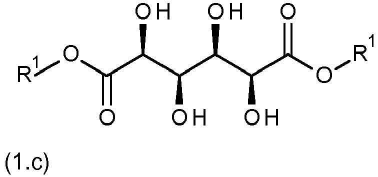
worin einer der Reste R¹ für C₁-C₂₄-Alkyl, C₂-C₂₄-Alkenyl mit 1, 2, 3 oder mehr als 3 C-C-Doppelbindungen oder für eine Gruppe der Formel -(R²-O)ₙ-R³ steht, worin jedes R² ausgewählt ist unter C₁-C₁₀-Alkylen, R³ für C₁-C₂₄-Alkyl oder C₂-C₂₄-Alkenyl mit 1, 2, 3 oder mehr als 3 C-C-Doppelbindungen steht und n für eine natürliche Zahl von 1 bis 1000 steht,
und der andere Rest R¹ für C₁-C₂₄-Alkyl, C₂-C₂₄-Alkenyl mit 1, 2, 3 oder mehr als 3 C-C-Doppelbindungen oder für eine Gruppe der Formel -(R²-O)ₙ-R³ steht, worin jedes R² ausgewählt ist unter C₁-C₁₀-Alkylen, R³ für C₁-C₂₄-Alkyl oder C₂-C₂₄-Alkenyl mit 1, 2, 3 oder mehr als 3 C-C-Doppelbindungen steht I und n für eine natürliche Zahl von 1 bis 1000 steht.

10. Wasch-, Reinigungs- oder Geschirrspülmittel oder Klarspüler, enthaltend wenigstens eine Verbindung der allgemeinen Formel (1), wie in Anspruch 9 definiert, und wenigstens ein davon verschiedenes Tensid.

11. Kosmetische oder pharmazeutische Zusammensetzung, enthaltend wenigstens eine Verbindung der allgemeinen Formel (1), wie in Anspruch 9 definiert, und wenigstens einen davon verschiedenen kosmetischen oder pharmazeutischen Wirkstoff und/oder Hilfsstoff.

12. Kosmetische Zusammensetzung nach Anspruch 9 in Form einer Creme, Mousse, Milch, Lotion, Mascara, Theaterfarbe, Seife von flüssiger bis fester Konsistenz, eines Schaums, Gels, Sprays, Stifts, Wasch-, Dusch- oder Badepräparats von flüssiger bis gelförmiger Konsistenz, Lidschattens, Eyeliners, Rouges, Puders oder Strips.

13. Verwendung wenigstens einer Verbindung der allgemeinen Formel (1), wie in Anspruch 9 definiert, als grenzflächenaktive Verbindung, bevorzugt als Tensid, Emulgator oder Schaumbildner.

14. Verwendung wenigstens einer Verbindung der allgemeinen Formel (1), wie in Anspruch 9 definiert, als grenzflächenaktive Verbindung in kosmetischen Mitteln, in pharmazeutischen Mitteln, in Wasch- und Reinigungsmitteln, in Geschirrspülmitteln und in Klarspülern, in Hygieneprodukten, in Pflanzenschutzmitteln, in Lacken, Beschichtungsmitteln, Klebstoffen, Lederbehandlungs- oder Textilbehandlungsmitteln, sowie bei der Erschließung und/oder Ausbeutung unterirdischer Erdöl- und/oder Erdgaslagerstätten.

## Claims

1. A method for preparing 2, 3, 4, 5-tetrahydroxyhexanedioic acid esters comprising at least one compound of the general formula (1), 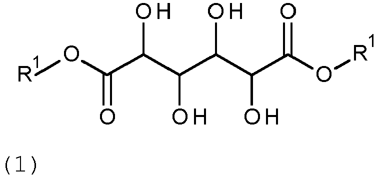 wherein the residues R¹ are mutually independently C₁-C₂₄-alkyl, C₂-C₂₄-alkenyl having 1, 2, 3 or more than 3 C-C double bonds, or are a group of the formula - (R²-O)ₙ-R³, in which each R² is selected from C₂-C₁₀-alkylene, R³ is C₁-C₂₄-alkyl or C₂-C₂₄-alkenyl having 1, 2, 3 or more than 3 C-C double bonds and n is a natural number from 1 to 1000,
by
a) providing a compound of the formula (2) 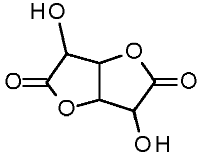 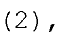 which is selected from compounds of the formulae (2.a), (2.b) and (2.c). 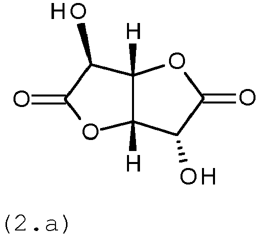 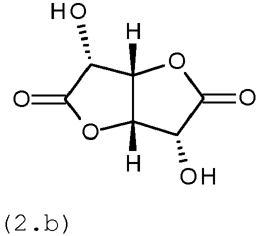 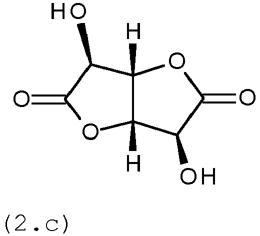 and
b) reacting the compound of the formula (2) provided in step a) with at least one compound of the general formula (3),
R¹-OH (3)
where R¹ is as defined above,
in the presence of at least one catalyst,
which is selected from compounds having at least one Lewis acidic, hard cation and/or at least one hard anion.

2. The method according to claim 1, wherein the 2, 3, 4, 5-tetrahydroxyhexanedioic acid esters additionally comprise at least one compound of the general formula (1') 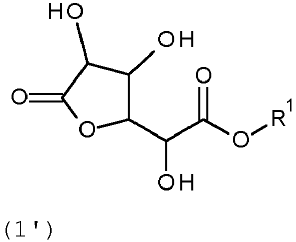 where R¹ is as defined in claim 1.

3. The method according to either of the preceding claims, wherein the total amount of compounds (3) in step b), based on the total amount of compound (2), is 0.5 to 100 molar equivalents, preferably 0.5 to 50 molar equivalents, particularly preferably 1 to 10 molar equivalents and especially preferably 1 to 4 molar equivalents.

4. The method according to any of the preceding claims wherein the the reaction in step b) is carried out at a temperature in the range of 10°C to 150°C, preferably in the range of 15°C to 90°C, and particularly preferably in the range of 20°C to 70°C.

5. The compound according to any of the preceding claims, wherein the catalyst in step b) is selected from compounds comprising at least one cation, which is selected from Al³⁺, Ti⁴⁺, Cu²⁺, Li⁺, Na⁺, K⁺, Be²⁺, Mg²⁺, Ca²⁺, Sr²⁺, Mn²⁺, Sc³⁺, Ga³⁺, In³⁺, La³⁺, Cr³⁺, Cr⁶⁺, Co³⁺, Fe³⁺, As³⁺, Ir³⁺, Si⁴⁺, Zr⁴⁺, Ce³⁺, Hf⁴⁺, Ge⁴⁺ VO²⁺, (CH₃) Sn²⁺, CH₃Sn³⁺, Fe²⁺, Co²⁺, Ni²⁺, Zn²⁺, Pb²⁺, Sn²⁺, Sb³⁺, Ru²⁺, Os²⁺, Rh³⁺, Bi³⁺, Cl³⁺, Sn⁴⁺, Lu³⁺, MoO³⁺, NO⁺ and WO⁴⁺.

6. The method according to any of the preceding claims, wherein the catalyst in step b) is selected from compounds comprising at least one anion, which is selected from alkanesulfonates, partially fluorinated alkanesulfonates, fully fluorinated alkanesulfonates, arenesulfonates, arylsulfonates, acetate, perchlorate, nitrate, fluoride, sulfate, carbonate, azide, bromide, phosphate, nitrite, sulfite, hydroxyl, O²⁻ and alkoxides.

7. The method according to any of the preceding claims, wherein the catalyst in step b) is selected from aluminum(III) methanesulfonate, aluminum(III) nonafluorobutanesulfonate, aluminum(III) para-toluenesulfonate, aluminum(III) trifluoromethanesulfonate, copper(I) methanesulfonate, copper(II) methanesulfonate, copper(I) trifluoromethanesulfonate, copper(II) trifluoromethanesulfonate, silver methanesulfonate, silver trifluoromethanesulfonate and tetra(isopropyl) titanate.

8. The method according to any of the preceding claims, wherein the reaction in step b) is carried out without addition of an external solvent or in the presence of at least one solvent different from the compounds R¹-OH, which is selected from aliphatic ethers, cyclic ethers, ketones, chlorinated hydrocarbons, aliphatic hydrocarbons, cycloaliphatic hydrocarbons, aromatic compounds and mixtures thereof.

9. A compound of the general formula (1), selected from
compounds of the formula (1.a), 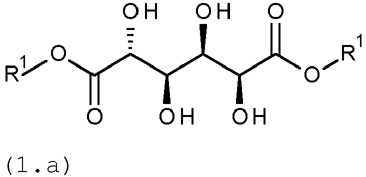
wherein one of the residues R¹ is C₁-C₂₄-alkyl, C₂-C₂₄-alkenyl having 1, 2, 3 or more than 3 C-C double bonds, or is a group of the formula -(R²-O)ₙ-R³, in which each R² is selected from C₂-C₁₀-alkylene, R³ is C₁-C₂₄-alkyl or C₂-C₂₄-alkenyl having 1, 2, 3 or more than 3 C-C double bonds and n is a natural number from 1 to 1000,
and the other residue R¹ is C₈-C₂₄-alkyl, C₂-C₂₄-alkenyl having 1, 2, 3 or more than 3 C-C double bonds, or is a group of the formula -(R²-O)ₙ-R³, in which each R² is selected from C₁-C₁₀-alkylene, R³ is C₁-C₂₄-alkyl or C₂-C₂₄-alkenyl having 1, 2, 3 or more than 3 C-C double bonds and n is a natural number from 1 to 1000;
compounds of the formula (1.b), 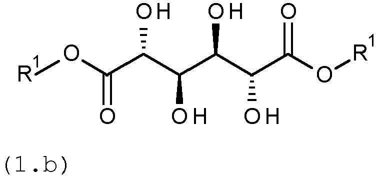
wherein one of the residues R¹ is C₁-C₂₄-alkyl, C₂-C₂₄-alkenyl having 1, 2, 3 or more than 3 C-C double bonds, or is a group of the formula -(R²-O)ₙ-R³, in which each R² is selected from C₁-C₁₀-alkylene, R³ is C₁-C₂₄-alkyl or C₂-C₂₄-alkenyl having 1, 2, 3 or more than 3 C-C double bonds and n is a natural number from 1 to 1000,
and the other residue R¹ is C₃-C₂₄-alkyl, C₂-C₂₄-alkenyl having 1, 2, 3 or more than 3 C-C double bonds, or is a group of the formula -(R²-O)ₙ-R³, in which each R² is selected from C₁-C₁₀-alkylene, R³ is C₁-C₂₄-alkyl or C₂-C₂₄-alkenyl having 1, 2, 3 or more than 3 C-C double bonds and n is a natural number from 1 to 1000; and
compounds of the formula (1.c), 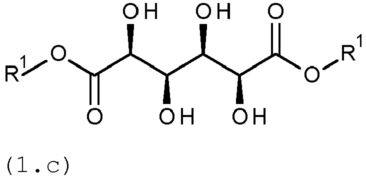
wherein one of the residues R¹ is C₁-C₂₄-alkyl, C₂-C₂₄-alkenyl having 1, 2, 3 or more than 3 C-C double bonds, or is a group of the formula -(R²-O)ₙ-R³, in which each R² is selected from C₁-C₁₀-alkylene, R³ is C₁-C₂₄-alkyl or C₂-C₂₄-alkenyl having 1, 2, 3 or more than 3 C-C double bonds and n is a natural number from 1 to 1000,
and the other residue R¹ is C₁-C₂₄-alkyl, C₂-C₂₄-alkenyl having 1, 2, 3 or more than 3 C-C double bonds, or is a group of the formula -(R²-O)ₙ-R³, in which each R² is selected from C₁-C₁₀-alkylene, R³ is C₁-C₂₄-alkyl or C₂-C₂₄-alkenyl having 1, 2, 3 or more than 3 C-C double bonds 1 and n is a natural number from 1 to 1000.

10. A washing, cleaning or dishwashing composition or rinse aid comprising at least one compound of the general formula (1), as defined in claim 9, and at least one surfactant different therefrom.

11. A cosmetic or pharmaceutical composition comprising at least one compound of the general formula (1), as defined in claim 9, and at least one cosmetic or pharmaceutical active ingredient and/or auxiliary different therefrom.

12. The cosmetic composition according to claim 9 in the form of a cream, mousse, milk, lotion, mascara, stage makeup, soap of liquid to solid consistency, a foam, gel, spray, stick, washing, showering or bathing preparations of liquid to gel consistency, eyeshadows, eyeliners, rouges, powders or strips.

13. The use of at least one compound of the general formula (1), as defined in claim 9, as surface-active compound, preferably as surfactant, emulsifier or foam former.

14. The use of at least one compound of the general formula (1), as defined in claim 9, as surface-active compound in cosmetic agents, in pharmaceutical agents, in washing and cleaning compositions, in dishwashing compositions and in rinse aids, in hygiene products, in plant protection agents, in lacquers, coating materials, adhesives, leather treatment compositions or textile care compositions, and also in the development and/or exploitation of underground mineral oil and/or natural gas deposits.

## Revendications

1. Procédé pour la préparation d'esters de l'acide 2,3,4,5-tétrahydroxyhexanedioïque comprenant au moins un composé de formule générale (1), 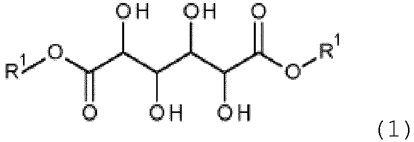 dans laquelle les radicaux R¹ représentent indépendamment l'un de l'autre C₁₋₂₄-alkyle, C₂₋₂₄-alcényle comportant 1, 2, 3 ou plus de 3 doubles liaisons C-C, ou représentent un groupe de formule -(R²-O)ₙ-R³, dans lequel chaque R² est choisi parmi C₂₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000,
dans lequel
a) on met à disposition un composé de formule (2) 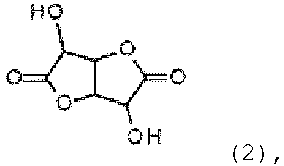 qui est choisi parmi les composés des formules
(2.a), (2.b) et (2.c) 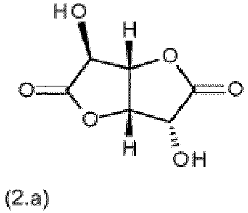 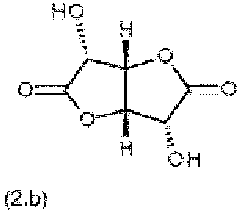 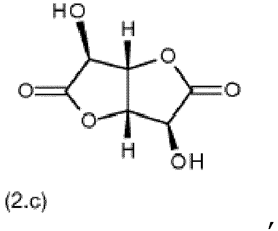 et
b) on met à réagir le composé de formule (2) mis à disposition dans l'étape a) avec au moins un composé de formule générale (3),
R¹-OH (3)
dans laquelle R¹ possède la signification définie ci-dessus,
en présence d'au moins un catalyseur,
qui est choisi parmi les composés qui présentent au moins un cation dur acide de Lewis et/ou au moins un anion dur.

2. Procédé selon la revendication 1, les esters de l'acide 2,3,4,5-tétrahydroxyhexanedioïque comprenant de plus au moins un composé de formule générale (1') 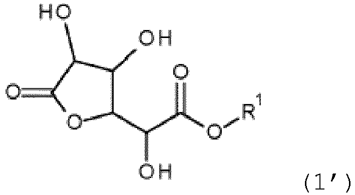 dans laquelle R¹ est défini tel que selon la revendication 1.

3. Procédé selon l'une quelconque des revendications précédentes, dans lequel dans l'étape b) la quantité totale des composés (3) est de 0,5 à 100 équivalents molaires, préférablement de 0,5 à 50 équivalents molaires, particulièrement préférablement de 1 à 10 équivalents molaires et tout particulièrement préférablement de 1 à 4 équivalents molaires, par rapport à la quantité totale du composé (2).

4. Procédé selon l'une quelconque des revendications précédentes, la transformation dans l'étape b) étant mise en oeuvre à une température dans la plage de 10 à 150°C, préférablement dans la plage de 15 à 90°C et particulièrement préférablement dans la plage de 20 à 70°C.

5. Procédé selon l'une quelconque des revendications précédentes, dans lequel dans l'étape b) le catalyseur est choisi parmi des composés, qui contiennent au moins un cation qui est choisi parmi Al³⁺, Ti⁴⁺, Cu²⁺, Li⁺, Na⁺, K⁺, Be²⁺, Mg²⁺, Ca²⁺, Sr²⁺, Mn²⁺, Sc³⁺, Ga³⁺, In³⁺, La³⁺, Cr³⁺, Cr⁶⁺, Co³⁺, Fe³⁺, As³⁺, Ir³⁺, Si⁴⁺, Zr⁴⁺, Ce³⁺, Hf⁴⁺, Ge⁴⁺, VO²⁺, (CH₃) Sn²⁺, CH₃Sn³⁺, Fe²⁺, Co²⁺, Ni²⁺, Zn²⁺, Pb²⁺, Sn²⁺, Sb³⁺, Ru²⁺, Os²⁺, Rh³⁺, Bi³⁺, Cl³⁺, Sn⁴⁺, Lu³⁺, MoO³⁺, NO⁺ et WO⁴⁺.

6. Procédé selon l'une quelconque des revendications précédentes, dans lequel dans l'étape b) le catalyseur est choisi parmi des composés qui contiennent au moins un anion qui est choisi parmi des alcanesulfonates, des alcanesulfonates partiellement fluorés, des alcanesulfonates entièrement fluorés, des arènesulfonates, des arylsulfonates, acétate, perchlorate, nitrate, fluorure, sulfate, carbonate, azoture, bromure, phosphate, nitrite, sulfite, hydroxyle, O²⁻ et des alcoolates.

7. Procédé selon l'une quelconque des revendications précédentes, dans lequel dans l'étape b) le catalyseur est choisi parmi le méthanesulfonate d'aluminium (III), le nonafluorobutanesulfonate d'aluminium (III), le para-toluènesulfonate d'aluminium (III), le trifluorométhanesulfonate d'aluminium (III), le méthanesulfonate de cuivre (I), le méthanesulfonate de cuivre (II), le trifluorométhanesulfonate de cuivre (I), le trifluorométhanesulfonate de cuivre (II), le méthanesulfonate d'argent, le trifluorométhanesulfonate d'argent et le titanate de tétraisopropyle.

8. Procédé selon l'une quelconque des revendications précédentes, la transformation dans l'étape b) étant mise en oeuvre sans ajout d'un solvant externe ou en présence d'au moins un solvant différent des composés R¹-OH, qui est choisi parmi les éthers aliphatiques, les éthers cycliques, les cétones, les hydrocarbures chlorés, les hydrocarbures aliphatiques, les hydrocarbures cycloaliphatiques, les composés aromatiques et des mélanges correspondants.

9. Composés de formule générale (1), choisis parmi des composés de formule (1.a), 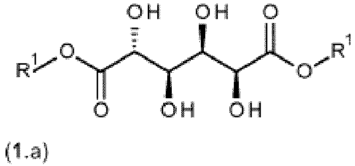
dans laquelle un des radicaux R¹ représente C₁₋₂₄-alkyle, C₂₋₂₄-alcényle comportant 1, 2, 3 ou plus de 3 doubles liaisons C-C, ou représente un groupe de formule -(R²-O)ₙ-R³, dans lequel chaque R² est choisi parmi C₂₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000,
et l'autre radical R¹ représente C₈₋₂₄-alkyle, C₂₋₂₄-alcényle comportant 1, 2, 3 ou plus de 3 doubles liaisons C-C, ou représente un groupe de formule - (R²-O)ₙ-R³, dans lequel chaque R² est choisi parmi C₁₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000 ;
des composés de formule (1.b), 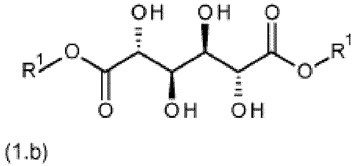
dans laquelle un des radicaux R¹ représente C₁₋₂₄-alkyle, C₂₋₂₄-alcényle comportant 1, 2, 3 ou plus de 3 doubles liaisons C-C, ou représente un groupe de formule -(R²-O)ₙ-R³, dans lequel chaque R² est choisi parmi C₁₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000,
et l'autre radical R¹ représente C₃₋₂₄-alkyle, C₂₋₂₄-alcényle comportant 1, 2, 3 ou plus de 3 doubles liaisons C-C, ou représente un groupe de formule - (R²-O)ₙ-R³, dans lequel chaque R² est choisi parmi C₁₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000 ; et
des composés de formule (1.c), 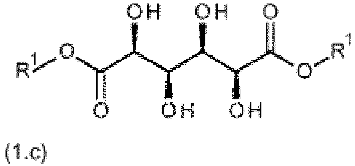
dans laquelle un des radicaux R¹ représente C₁₋₂₄-alkyle, C₂₋₂₄-alcényle comportant 1, 2, 3 ou plus de 3 doubles liaisons C-C, ou représente un groupe de formule -(R²-O)ₙ-R³, dans lequel chaque R² est choisi parmi C₁₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000,
et l'autre radical R¹ représente C₁₋₂₄-alkyle, C₂₋₂₄-alcényle comportant 1, 2, 3 ou plus de 3 doubles liaisons C-C, ou représente un groupe de formule - (R²-O)ₙ-R³, dans lequel chaque R² est choisi parmi C₁₋₁₀-alkylène, R³ représente C₁₋₂₄-alkyle ou C₂₋₂₄-alcényle comportant 1, 2, 3 ou plus de 3 doubles liaisons C-C et n représente un nombre naturel de 1 à 1000.

10. Détergent, produit de nettoyage ou produit de lavage de la vaisselle ou produit de rinçage contenant au moins un composé de formule générale (1), tel que défini selon la revendication 9, et au moins un tensioactif différent de celui-ci.

11. Composition cosmétique ou pharmaceutique contenant au moins un composé de formule générale (1) tel que défini selon la revendication 9, et au moins une substance active et/ou un adjuvant cosmétique ou pharmaceutique différent de celui-ci.

12. Composition cosmétique selon la revendication 9 sous forme d'une crème, d'une mousse, d'un lait, d'une lotion, d'un mascara, d'un fard de théâtre, d'un savon de consistance liquide à solide, d'une écume, d'un gel, d'un spray, d'un crayon, d'un produit de nettoyage, d'un produit de douche, d'un produit de bain de consistance liquide à gélifiée, d'un fard à paupières, d'un eye-liner, d'un rouge à lèvres, d'une poudre ou d'un strip.

13. Utilisation d'au moins un composé de formule générale (1), tel que défini selon la revendication 9, en tant que composé tensioactif, préférablement en tant que tensioactif, émulsifiant ou agent moussant.

14. Utilisation d'au moins un composé de formule générale (1), tel que défini selon la revendication 9, en tant que composé tensioactif dans des produits cosmétiques, dans des produits pharmaceutiques, dans des détergents et des produits de nettoyage, dans des produits de lavage de la vaisselle ou des produits de rinçage, dans des produits d'hygiène, dans des produits phytosanitaires, dans des laques, des produits de revêtement, des adhésifs, des produits de traitement du cuir ou des produits de traitement de textiles, ainsi que dans l'aménagement et/ou l'exploitation de champs pétrolifères et/ou de gaz naturel souterrains.
